(19) Europäisches Patentamt
European Patent Office
Office européen des brevets

(11) EP 0 770 061 B1

(12) EUROPÄISCHE PATENTSCHRIFT

(45) Veröffentlichungstag und Bekanntmachung des Hinweises auf die Patenterteilung:
**07.04.1999 Patentblatt 1999/14**

(21) Anmeldenummer: **95924331.2**

(22) Anmeldetag: **28.06.1995**

(51) Int Cl.[6]: **C07C 319/28**, C07C 319/20, C07C 323/60, A23K 1/16

(86) Internationale Anmeldenummer:
**PCT/EP95/02515**

(87) Internationale Veröffentlichungsnummer:
**WO 96/01809 (25.01.1996 Gazette 1996/05)**

(54) **VERFAHREN ZUR GEWINNUNG VON 2-HYDROXY-4-METHYLTHIOBUTTERSÄURE (MHA), MHA UND DESSEN VERWENDUNG**

PROCESS FOR OBTAINING 2-HYDROXY-4-METHYL THIOBUTYRIC ACID (MHA), MHA AND ITS USE

PROCEDE D'OBTENTION D'ACIDE 2-HYDROXY-4-METHYLTHIOBUTYRIQUE (MHA), MHA OBTENU ET SON UTILISATION

(84) Benannte Vertragsstaaten:
**BE DE ES FR GB GR IT NL PT**

(30) Priorität: **11.07.1994 DE 4424043**

(43) Veröffentlichungstag der Anmeldung:
**02.05.1997 Patentblatt 1997/18**

(73) Patentinhaber: **Degussa Akfiengesellschaft
60311 Frankfurt (DE)**

(72) Erfinder:
- **HASSEBERG, Hans-Albrecht
D-63584 Gründau (DE)**
- **HUTHMACHER, Klaus
D-63571 Gelnhausen (DE)**
- **TANNER, Herbert
D-63457 Hanau (DE)**
- **HÄFNER, Volker
D-63505 Langenselbold (DE)**
- **HEINZEL, Harald
D-60488 Frankfurt am Main (DE)**

(56) Entgegenhaltungen:
**EP-A- 0 142 488**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 770 061 B1

Printed by Jouve, 75001 PARIS (FR)

## Beschreibung

Die Erfindung betrifft ein Verfahren zur Gewinnung von 2-Hydroxy-4-methylthiobuttersäure (MHA) nach dem Oberbegriff des Anspruchs 1, gemäß der Erfindung erhältliches MHA sowie die Verwendung des nach diesem Verfahren hergestellten MHA's.

Insbesondere betrifft die Erfindung ein verbessertes, neuartiges Isolationsverfahren von MHA in sehr hoher Konzentration und Reinheit aus einem bei der Herstellung resultierenden Reaktionsgemisch.

2-Hydroxy-4-methylthiobuttersäure (MHA) ist das Hydroxyanaloge der essentiellen Aminosäure Methionin in racemischer Form und ist wie diese ein wichtiger Zusatzstoff in der Tierernährung. In der Geflügelaufzucht zeigt MHA ähnliche wachstumsstimulierende Eigenschaften wie die dafür bekannte Aminosäure. Aber auch in anderen Bereichen der Tierernährung findet das Additiv zunehmendes Interesse.

Eingesetzt wird MHA meist in Form wässriger Konzentrate, wobei diese neben dem Monomeren noch einen gewissen Anteil an Oligomeren, hauptsächlich die di- und trimeren linearen Estersäuren enthalten. Der Gehalt an diesen Oligomeren hängt von den Herstellungsbedingungen und der gewählten Konzentration ab. Wegen ihres geringeren nutritiven Wirkungsgrades und des ungünstigen Einflusses auf die Fließeigenschaften infolge Viskositätserhöhung ist es jedoch wünschenswert, ihren prozentualen Anteil möglichst niedrig zu halten. Handelsübliche Formulierungen weisen bei einer Gesamtkonzentration von 88 - 90 Gew.-% bis zu 24 Gew.-%, entsprechend ca. 27 Mol-%, in der Summe an Oligomeren auf, entsprechend einem Monomeren/Oligomeren-Verhältnis von ~ 3 : 1.

Bekannt ist auch die Verwendung des Calziumsalzes und des gemischten Calziumammoniumsalzes des MHA als Tierfutterzusatz. Die Herstellung dieser Salze ist aber mit höheren Gestehungskosten verbunden. Außerdem lassen sie sich als pulverförmige Feststoffe weniger leicht in die Futtermittel formulierung einmischen als die leicht zu versprühenden wässrigen Konzentrate der freien Säure mit niedrigem Oligomerenanteil.

Der Syntheseweg zum MHA besteht aus 3 Reaktionen.

Das allgemeine Verfahren zur Herstellung von MHA geht von 3-Methylthiopropionaldehyd, auch als Methylmercaptopropionaldehyd oder MMP bezeichnet, aus, der mit Cyanwasserstoff zum 2-Hydroxy-4-methylthiobutyronitril, auch als MMP-Cyanhydrin oder MMP-CH bezeichnet, umgesetzt wird (Gleichung I).

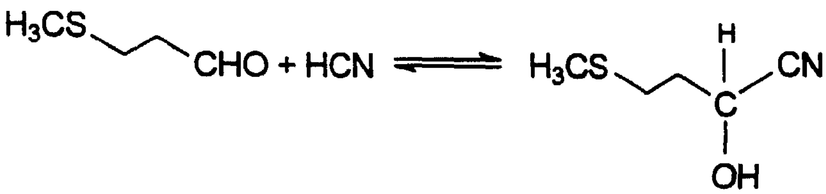

(I)

Das entstandene MMP-Cyanhydrin wird anschließend üblicherweise mit starken Mineralsäuren über die Zwischenstufe des 2-Hydroxy-4-methylthiobutyramids, auch als MHA-Amid bezeichnet (Gleichung II),

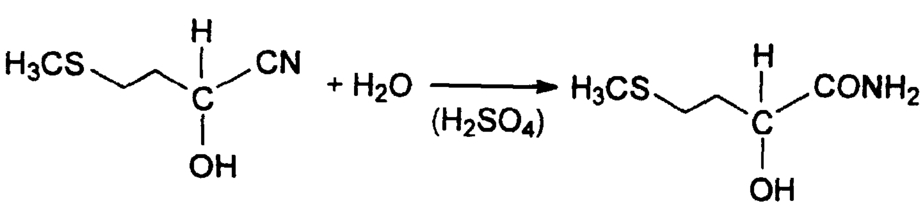


(II)

zum Methioninhydroxyanalogen (MHA) hydrolysiert (Gleichung III).

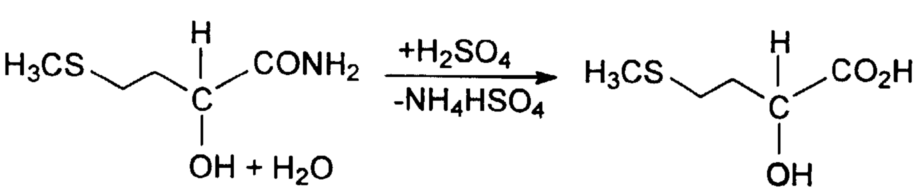


(III)

Diese Hydrolyse kann sowohl ein- als auch zweistufig durchgeführt werden, wobei unter "Stufen" zu verstehen

ist, daß zur Hydrolyse des MMP-CH's entweder einmal oder zweimal Mineralsäure und/oder Wasser zugesetzt wird, d. h. die Anzahl der Stufen entspricht der Anzahl der Zugabevorgänge.

Eine zweistufige Arbeitsweise ausgehend von MMP-Cyanhydrin beschreiben die US-Patente 2 745 745, 2 938 053 und 3 175 000. Dort wird zunächst bei relativ niedrigen Temperaturen mit konzentrierter Mineralsäure z. B. mit 50 - 85 %iger Schwefelsäure das Cyanhydrin zum MHA-Amid umgesetzt, worauf dann nach Zusatz von Wasser bei erhöhter Temperatur die Hydrolyse zum MHA weitergeführt wird. Durch Behandlung des Verseifungsgemischs mit Calziumhydroxid oder -carbonat erhält man hieraus das Calzium- bzw. Calziumammoniumsalz des MHA und als Koppelprodukt Calziumsulfat. Zur Verminderung des Zwangsanfalls wertloser Nebenprodukte empfehlen die beiden erstgenannten Patentschriften das Hydrolyseagenz Schwefelsäure im unterstöchiometrischen Verhältnis zum MMP-Cyanhydrin, beispielsweise 0,55 - 0,8 : 1 einzusetzen. Auch das britische Patent 722 024, welches die gleichartige Bildung der MHA-Salze ausgehend von MHA-Amid beschreibt, impliziert die zweistufige Arbeitsweise.

Ebenfalls der Zweistufenhydrolyse bedienen sich die in den europäischen Patentschriften 0 142 488 (mit Schwefelsäure) und 0 143 100 (mit Mineralsäure) publizierten Verfahren, die die Gewinnung von MHA in flüssiger Form, also hochkonzentrierter wässriger Lösungen, zum Gegenstand haben. Diese erhält man nach der, unter definierten Konzentrations- und Temperaturbedingungen über die Amidstufe mit überschüssiger Mineralsäure durchgeführten Hydrolysereaktion, mit Hilfe einer Lösungsmittelextraktion, wobei bestimmte, mit Wasser partiell mischbare Lösungsmittel zur Anwendung kommen.

Gemäß den Angaben in diesen Patentschriften ist das kennzeichnende Merkmal des dort beschriebenen Verfahrens in der Gewinnung des MHA's aus der Extraktionslösung zu sehen, welche so durchgeführt wird, daß die Gewinnung das Entfernen des organischen Lösungsmittels in Anwesenheit von zumindest etwa 5 Gew.-% Wasser, bezogen auf den verbleibenden Extrakt (MHA), umfaßt. MHA wird aus der Extraktionslösung durch Destillation gewonnen (siehe Beispiele), wobei die Dampfdestillation bevorzugt ist. Durch Entfernung des Lösungsmittels aus der Extraktionslösung während der Dampfdestillation ist der erhaltene Ablauf eine Mischung aus MHA und Wasser. Die Dampfdestillation wird also demzufolge so geführt, daß der Ablauf mindestens 5 Gew.-% Wasser enthält.

An anderer Stelle führen die in Rede stehenden Patentschriften aus, daß die Kolonnenbedingungen bei der Destillation so reguliert werden, daß überall in der Kolonne, zumindest jedoch in der Bodenfraktion, die flüssige Phase 5 Gew.-% Wasser aufweist.

Daraus folgt, daß ohne Gegenwart einer ausreichenden Menge Wasser während der Gewinnung des MHA aus der Extraktionslösung, die zunehmende Bildung unerwünschter Nebenprodukte (Dimere und Oligomere) zu befürchten ist.

Weiterhin dient der Wasserdampf in der Destillation als treibendes Agens zur restlosen Entfernung des Extraktionsmittels aus der MHA-Lösung, z. B. durch Bildung eines tiefsiedenden Azeotropgemisches mit dem entsprechenden Extraktionsmittel.

Konzentrierte MHA-Lösungen ohne Zuhilfenahme eines Lösungsmittels gewinnt man gemäß der US-Patentschrift 3 773 927 mittels Zweistufenhydrolyse des MMP-Cyanhydrins mit wässriger Salzsäure im Überschuß, nachfolgender Konzentrierung der Verseifungsmischung und Abtrennung des auskristallisierten Ammoniumchlorids. Die so erhaltenen MHA-Konzentrate sind jedoch reich an Oligomeren und schwarz verfärbt. Auch das abgetrennte Ammoniumchlorid ist stark verunreinigt.

Nach der US-Patentschrift 4 353 924 wird nach der salzsauren zweistufigen Hydrolyse der Überschuß an Mineralsäure mit Ammoniak oder anderen alkalischen Stoffen neutralisiert. Man erhält so konzentrierte MHA-Lösungen mit geringen korrosiven Eigenschaften.

Im US-Patent 4 310 690 wird ein Verfahren beschrieben, bei welchem nach der Hydrolyse mit Salzsäure unter genau definierten Bedingungen mit Natronlauge neutralisiert und das Ammonchlorid in Kochsalz und Ammoniak umgewandelt wird. Bei der anschließenden Behandlung mit Ätzkalk erhält man das MHA-Calziumsalz als Aufschlämmung in einer praktisch gesättigten Kochsalzlösung. Nach der Fest-Flüssigtrennung werden die Filtrate größtenteils zur Bereitung der Ätzkalkaufschlämmungen wieder zurückgeführt. Auf diese Weise vermindert man die Abwasserbelastung und vermeidet die Coproduktion wertloser oder umweltbelastender Ballaststoffe.

Einstufige Hydrolyseverfahren sind in der Patentliteratur ebenfalls beschrieben. So ist das Verfahren gemäß der britischen Patentschrift 915 193 auf die Gewinnung des MHA-Calziumsalzes gerichtet, wobei nach der Verseifung des MMP-Cyanhydrins mit verdünnter Schwefelsäure im Überschuß das gebildete MHA mittels Extraktion mit höher siedenden Ethern von der Verseifungslösung abgetrennt und durch nachfolgende Behandlung des Extraktes mit Calziumhydroxyd das MHA-Calziumsalz gewonnen wird. Die bei diesem kontinuierlichen Verfahren vorgesehene Rückführung des wässrigen Raffinats in die Verseifungsstufe führt allerdings zu einer Akkumulierung der anorganischen Begleitstoffe.

In der europäischen Patentschrift 0 330 527 wurde ein weiteres einstufiges Hydrolyseverfahren mit Schwefelsäure als Verseifungsagenz publiziert, das ohne Lösungsmittel auskommt und direkt zu konzentrierten wässrigen MHA-Lösungen führt, wobei als Coprodukt kristallines Ammoniumsulfat in verkaufsfähiger Form erhalten wird. Dieses Ziel wird erreicht, indem man das Verseifungsgemisch mit Ammoniumhydroxidlösung soweit neutralisiert, daß die

überschüssige Mineralsäure und das entstandene Ammoniumbisulfat in das neutrale Sulfat überführt werden, wobei zwei flüssige Phasen entstehen, die ihrerseits getrennt und eingedampft werden, um flüssiges MHA einerseits und kristallines Ammoniumsulfat andererseits zu gewinnen. Dabei werden die verschiedenen Filtrations- und Rückführungsschritte so kombiniert, daß praktisch kein Produkt verloren geht und kein mit Salz belastetes Abwasser entsteht. Das resultierende MHA ist von ähnlicher Qualität wie das nach der EP 0 142 488 erhaltene Produkt.

Jedoch auch dieses umweltschonende Verfahren weist diverse Nachteile auf. Wie die Anmelderin der vorliegenden Erfindung beim Nacharbeiten dieses Verfahrens feststellte, müssen zum einen, bedingt durch die vergleichsweise höhere Verdünnung der Schwefelsäure (20 - 50 %), deutlich höhere Säureüberschüsse als angegeben verwendet werden, um zu einem vollständigen Cyanhydrin-Umsatz zu kommen. Auch muß zur Vermeidung von Salzausscheidungen während der Neutralisation in höherer Verdünnung gearbeitet werden, um die beiden flüssigen Phasen sauber trennen zu können. Zum anderen ist das isolierte Ammoniumsulfat von klebriger Konsistenz und mit einem intensiven Geruch behaftet, so daß eine Nachbehandlung wie z. B. eine Waschfiltration oder Umkristallisation unumgänglich erscheint, wodurch das Verfahren zusätzlich verteuert wird. Auch ist das Verfahren in den Eindampfschritten - anders als postuliert - energieaufwendiger als das zum Vergleich herangezogene Verfahren der EP-A 0 142 488. Kostenintensiv und apparativ sehr aufwendig ist außerdem das mit zwei getrennten Strängen ausgestattete Feststoffhandling mit Filtration/Zentrifugation sowie der im Fließdiagramm nicht angegebenen Trocknung des Ammoniumsulfats.

Angesichts des hierin angegebenen Stands der Technik sowie den mit den bekannten Verfahren verbundenen Nachteilen ist es Aufgabe der Erfindung, ein weiteres Verfahren zur Herstellung von 2-Hydroxy-4-methylthiobuttersäure (MHA) gemäß der eingangs erwähnten Gattung anzugeben, welches hinsichtlich der Aufarbeitung der Reaktionsprodukte möglichst einfach und kostengünstig sein soll und ein möglichst hochkonzentriertes Produkt mit möglichst geringem Gehalt an Dimeren, Oligomeren und Nebenprodukten gestatten soll. Aufgabe der Erfindung ist auch die Angabe eines verbesserten MHA's sowie dessen Verwendung.

Gelöst werden diese sowie weitere nicht im einzelnen angegebene Aufgaben mit einem Verfahren der eingangs genannten Gattung, das die Merkmale des kennzeichnenden Teils des Anspruches 1 aufweist.

Vorteilhafte Verfahrensvarianten werden in den von Anspruch 1 abhängigen Verfahrensansprüchen unter Schutz gestellt. In Bezug auf ein verbessertes MHA stellt Anspruch 8 eine Lösung des erfindungsgemäßen Problems dar. Neue Verwendungen sind Gegenstand der Ansprüche 9 und 10.

Dadurch, daß die Eindampfung der Extraktionslösung zur Gewinnung des MHA als Produkt (verbleibender Extrakt) so geführt wird, daß der verbleibende Extrakt weniger als 4 Gew.-%, bevorzugt weniger als 2 Gew.-%, Wasser aufweist, wird erfindungsgemäß ein Verfahren zur Verfügung gestellt, welches die Herstellung von flüssigem MHA in hervorragender Qualität gestattet und welches insbesondere in nicht vorhersehbarer Weise für ein hochkonzentriertes flüssiges MHA einen geringen Oligomeren- und Dimerenanteil ergibt. Dabei ist es angesichts der im Stand der Technik bekannten Verfahren, vor allem der beiden europäischen Patentschriften 0 142 488 und 0 143 100, als mehr als überraschend zu bezeichnen, daß dies mit geringerem Wasseranteil erreicht werden kann, als dies etwa der Fachmann den genannten Patenten entnehmen kann. Im Rahmen der Erfindung hat es sich weiterhin unvorhersehbar gezeigt, daß nahezu 100 %ige MHA-Lösung erhältlich sind, die ohne deutlichen Qualitätsverlust transportierbar und dann auf gewünschte Konzentrationen mit geeigneten Zusätzen oder Verdünnungsmitteln einstellbar sind. Diese Befunde stellen enorme wirtschaftliche Vorteile für ein großtechnisches Verfahren dar.

Die vorliegende Erfindung befaßt sich also in einem wichtigen Aspekt, insbesondere mit der Eindampfung einer Extraktionslösung, wie sie aus einer Flüssig-Flüssig-Extraktion eines Reaktionsgemisches erhältlich ist, das beispielsweise durch Hydrolyse von MMP-CH mit Schwefelsäure erhalten wird.

Die für die Eindampfung zur Gewinnung des MHA verwendete Extraktionslösung kann nach dem Fachmann geläufigen Verfahren aus dem Reaktionsgemisch, beispielsweise durch Extraktion, gewonnen werden. Hierfür sollte das zur Extraktion eingesetzte organische Lösungsmittel im wesentlichen mit Wasser nicht mischbar sein. Allerdings ist eine partielle Mischbarkeit des organischen Lösungsmittels mit Wasser tolerierbar. Unter den für die Stofftrennung in der Flüssig/Flüssig-Extraktion in Frage kommenden Lösungsmitteln gibt es eine Vielzahl, die die Bedingungen der chemischen Indifferenz und einer geringen Löslichkeit für Wasser erfüllen. Im allgemeinen ist es bevorzugt, daß die Löslichkeit von Wasser im Lösungsmittel nicht größer als ca. 15 Gew.-%, vorzugsweise nicht größer als 10 Gew.-% bei Raumtemperatur ist. Unter den geeigneten Lösungsmitteln sind solche bevorzugt, die einen Siedepunkt von zwischen ca. 60 °C und ca. 200 °C, bevorzugt zwischen ca. 70 °C und 150 °C, aufweisen. Der Verteilungskoeffizient zwischen dem Lösungsmittel, welches das extrahierte MHA enthält, und dem wässrigen Raffinat, welches nach dem Kontaktieren von Lösungsmittel und MHA-Hydrolysat zurückbleibt, sollte mindestens bei ca. 2 für das MHA im Gleichgewicht liegen. Vorzugsweise ist dieser Verteilungskoeffizient wenigstens = 5. Auch soll der Verteilungskoeffizient für MHA im Gleichgewicht zwischen Extraktionslösung und Waschwasser ca. 1,0 nicht unterschreiten. Weiterhin soll das Lösungsmittel eine niedrige Toxizität aufweisen.

Eine Reihe von Ketonen, Aldehyden und Carbonsäureestern sind besonders als Lösungsmittel für die Extraktion geeignet. Besonders bevorzugte Lösungsmittel sind Ketone mit verhältnismäßig geringem Molekulargewicht,

wie z. B. Methyl-n-propylketon, Methylethylketon, Methylamylketon, Methylisoamylketon, Methylisobutylketon, Ethylbutylketon und Diisobutylketon. Ebenfalls gut geeignete Lösungsmittel für die Extraktion sind Aldehyde, wie z. B. n-Butyraldehyd, und Ester, wie z. B. Ethylacetat, n-Butylacetat, n-Propylacetat und Isopropylacetat. Es können auch Alkohole verwendet werden, obwohl diese aufgrund ihrer wechselseitigen Löslichkeit mit Wasser, einer langsamen Phasenseparierung und der Tendenz mit dem MHA zu reagieren, weniger bevorzugt sind.

Die Extraktion selbst kann grundsätzlich kontinuierlich oder intermittierend durchgeführt werden. Für eine diskontinuierliche Verfahrensweise eignet sich beispielsweise ein Rührkessel. Bevorzugt jedoch wird die Extraktion in einer kontinuierlichen Gegenstrom-Extraktionsanlage durchgeführt, welche eine zur Beschleunigung des Massentransfers zwischen Lösungsmittel und wäßriger Phase ausgebildete Extraktionszone aufweist.

So ist es beispielsweise vorteilhaft, die Extraktion in einer Kaskade von kontinuierlichen Gegenstrom-Mischer-Abscheidern, einer Füllkörperkolonne, einer Siebbodenkolonne, bevorzugt als Pulsationskolonne oder Kolonne mit bewegten Sieben, einer Drehscheibenkolonne oder einem Zentrifugalextraktor durchzuführen. Bei einer besonders bevorzugten Ausführungsform wird die Extraktion in einer Siebbodenkolonne für Flüssig/Flüssig-Extraktion durchgeführt. Intermittierende oder impulsförmige Ströme, obwohl zyklisch also nicht kontinuierlich im Sinne von schneller Strömungsrate, werden im Zusammenhang mit der vorliegenden Offenbarung als "kontinuierlich" angesehen.

Der Extraktionsvorgang wird vorzugsweise reguliert, um die Lösungsmittelphase in der Extraktionszone als die kontinuierliche Phase herzustellen und aufrechtzuerhalten.

Um den Salzgehalt des Endproduktes auf ein Minimum zu bringen, wird der Extrakt vorzugsweise mit Wasser gewaschen. In einem kontinuierlichen Gegenstromextraktionssystem kann der Extrakt durch Vermischen mit Wasser an einer Stelle stromaufwärts, in Bezug auf die Richtung der organischen Strömung, der Stelle, an der Hydrolysat in das Flüssig/Flüssigextraktionssystem eingebracht wird, gewaschen werden. So werden beispielsweise in einer vertikalen Kolonne unter Verwendung eines Lösungsmittels, dessen spezifisches Gewicht bevorzugt weniger als 1 beträgt, Lösungsmittel in die Kolonne an einer Stelle unterhalb der Zufuhrstelle, an der wässrige Hydrolysatlösung eingebracht wird, und Waschwasser in die Kolonne an einer Stelle oberhalb des Zufuhrpunktes der Hydrolysatlösung eingebracht. Bei einer bevorzugten Ausführungsform wird das Lösungsmittel mit einer Rate von etwa 0,5 bis 0,6 Gew.-Teilen pro Gewichtseinheit Hydrolysat zugeführt, womit ein Extrakt mit einem spezifischen Gewicht von etwa 0,92 bis 0,97 und einem MHA-Gesamt-Gehalt (MHA-Gesamt = Summe MHA-Monomeres, -Dimeres + Oligomere + ggf. MHA-Amid) von 35 bis 40 Gew.-% erhalten wird.

Die Produktivität des Extraktionsvorganges wird durch Arbeiten bei einer etwas erhöhten Temperatur, um für die Lösungsmittelphase innerhalb des Extraktionssystems eine relativ niedrige Viskosität vorzusehen, erhöht. Das Arbeiten bei einer Temperatur im Bereich von etwa 50 bis etwa 80 °C ergibt auch eine gerade noch günstige Wirkung auf den MHA-Verteilungskoeffizienten zwischen der organischen und der wässerigen Phase.

Im Rahmen der Erfindung kann das MHA aus der Extraktionslösung wie bereits erwähnt durch Eindampfung gewonnen werden. In besonders bevorzugter erfindungsgemäßer Ausführungsform wird das organische Lösungsmittel bei der Eindampfung mit einem Aggregat entfernt, welches eine kurze Verweilzeit der Extraktionslösung in einer Eindampfungsstufe gestattet. Besonders bevorzugt wird das organische Lösungsmittel bei der Eindampfung daher mit einem Fallfilmverdampfer, Dünnschichtverdampfer und/oder Kurzwegverdampfer oder unter Mithilfe eines solchen Aggregats aus der Extraktionslösung abgetrennt.

Unter dem Begriff "Mithilfe eines solchen Aggregats" wird im Rahmen der Erfindung verstanden, daß die genannten Aggregate mit kurzer Verweilzeit der Extraktionslösung auch mit dem Fachmann bekannten Einrichtungen zur Abtrennung des Lösungsmittels aus Extraktionslösungen kombiniert werden können. Hierbei muß es sich bei den, zur Kombination verwendeten Aggregaten nicht unbedingt um solche, mit einer kurzen Verweilzeit handeln. Genannt werden können an dieser Stelle u. a. Destillationskolonnen, die wahlweise auch zur Einleitung von Dampf oder anderen geeigneten Strippmitteln ausgerüstet sein können. Auch Kombinationen, die mehrere der aufgeführten Aggregate mit kurzer Verweilzeit umfassen, sind möglich.

In vorteilhafter Abwandlung des erfindungsgemäßen Verfahrens ist es bevorzugt, die Eindampfung der Extraktionslösung so zu führen, daß ein möglichst geringer Restlösungsmittelgehalt eingestellt wird. Dies wird beispielsweise durch Kombination mehrerer oben genannter Aggregate mit einer Strippstufe, welche als zusätzliches Aggregat oder integriert in die vorgenannten Aggregate im Verdampfersystem enthalten sein kann, wie z. B. beim direkten Eintrag des Strippmediums in einen solchen Verdampfer, erreicht.

Die spezifischen Bedingungen für die Eindampfung variieren notwendigerweise mit dem für die Verwendung bei der Extraktion verwendeten speziellen Lösungsmittel. Grundsätzlich ist es für die Eindampfung unter Verwendung eines Abtrennaggregats mit kurzer Verweilzeit der Extraktionslösung bevorzugt, daß der Druck während der Eindampfung nicht mehr als 600 mbar, bevorzugt 400 mbar und besonders bevorzugt 200 mbar, beträgt.

Die bei der Eindampfung anzuwendende Temperatur ist im Regelfall ebenfalls vom abzutrennenden Lösungsmittel abhängig. Es wird jedoch angestrebt, und ist daher im Rahmen der Erfindung auch besonders bevorzugt, daß die Temperatur während der Eindampfung nicht höher als 150 °C ist. Wird diese Temperatur sehr deutlich überschritten, so kann es zur thermischen Schädigung des angestrebten Produktes kommen. Hierbei versteht sich unter der Temperatur während der Eindampfung nicht notwendigerweise die Kontakt temperatur des Produktes mit der Oberfläche des zur kurzzeitigen Berührung mit dem Produkt ausgestatteten Verdampfungsaggregates. Vielmehr ist mit der Temperatur während der Eindampfung die durchschnittliche Temperatur im Eindampfungsaggregat gemeint. Die Temperatur an der Oberfläche des Eindampfungsaggregates kann im Zweifelsfalle sehr viel höher liegen, als die 150 °C. Entscheidend ist die Kürze der Kontaktzeit bei den verwendeten Eindampfungsaggregaten. Hierdurch wird eine thermische Schädigung vermieden, selbst wenn die Kontakttemperatur deutlich über 150 °C liegen sollte.

Bezüglich der Temperaturverteilung hat es sich im Rahmen der Erfindung herausgestellt, daß es für die Produktqualität von besonderem Vorteil ist, wenn die Temperatur des verbleibenden Extraktes unmittelbar am Austritt aus dem Eindampfungsaggregat zwischen 30 und 100 °C, bevorzugt 50 bis 95 °C und besonders bevorzugt 70 bis 90 °C, beträgt.

Wie bereits erwähnt, ist die Verweilzeit des verbleibenden Extraktes mit ausschlaggebend für die Qualität und Zusammensetzung des angestrebten MHA-Produkts. In vorteilhafter Weiterbildung des erfindungsgemäßen Verfahrens beträgt die Verweilzeit des verbleibenden Extraktes in der Eindampfung nicht länger als 1,5 h. Dies bezieht sich auf die Verweilzeit im gesamten Eindampfungssystem, welches zumindest eine Eindampfungsstufe mit sehr kurzer Verweilzeit aufweist. Die Verweilzeit im Aggregat mit sehr kurzer Verweilzeit ist entgegen der für die Gesamtverweilzeit maximal angegebenen 1,5 h eher im Minutenbereich oder darunter anzusiedeln. Auf jeden Fall ist es im Rahmen der Erfindung bevorzugt, für den Fall, daß die Eindampfung lediglich aus einem Dünnschichtverdampfer und/oder Fallfilmverdampfer und/oder Kurzwegverdampfer besteht, daß die Verweilzeit in diesen Aggregaten nicht länger als 1 h, bevorzugt 40 min beträgt.

In einem weiteren Aspekt verbessert das erfindungsgemäße Verfahren zusätzlich zur Isolierung des MHA's aus dem durch Hydrolyse mit Schwefelsäure erhaltenen Reaktionsgemisch auch die Hydrolyse des MMP-CH's selbst. So wird in erfindungsgemäß bevorzugter Ausführungsform die Hydrolyse des MMP-CH so geführt, daß in einer ersten Stufe das MMP-CH mit 60 - 85 gew.-%iger, bevorzugt mit 65 - 80 gew.-%iger Schwefelsäure im Molverhältnis MMP-CH zu $H_2SO_4$ von 1,0:0,5 bis 1:1,05, vorzugsweise 1:0,6 bis 1:0,8 bei Temperaturen von 30 - 90 °C, vorzugsweise 50 - 70 °C unter Erhalt von im wesentlichen MHA-Amid hydrolysiert wird. Hierbei entsteht im wesentlichen aus dem MMP-Cyanhydrin das MHA-Amid, wobei die entstehende Mischung weiterhin im wesentlichen frei von nicht umgesetzten MMP-Cyanhydrin ist. Mit anderen Worten bedeutet dies, daß die Hydrolyse nahezu quantitativ verläuft.

Weiterhin ist es für die Erfindung besonders vorteilhaft, wenn die Hydrolyse des in der ersten Stufe erhaltenen MHA-Amids in einer zweiten Stufe durch Zugabe von Wasser und geringstenfalls weiterer Schwefelsäure bis zur stöchiometrischen Obergrenze, bevorzugt jedoch ohne weitere Zugabe von weiterer $H_2SO_4$ bei Temperaturen bis 140 °C, vorzugsweise zwischen 100 und 140 °C, vorzugsweise unter Rückflußbedingungen, ausgeführt wird, um die Hydrolyse des MHA-Amids zum MHA zu vervollständigen. Grundsätzlich kann die zweite Hydrolysestufe auch bei Temperaturen von unter 100 °C, beispielsweise bis zu Obergrenzen von Temperaturen von 90 - 100 °C, durchgeführt werden. In diesem Falle ist jedoch der Zusatz weiterer Schwefelsäure bis maximal zur stöchiometrischen Obergrenze nötig. Um die Hydrolyse des MHA-Amids zum angestrebten MHA ohne Zusatz weiterer Schwefelsäure nur unter Zugabe von Wasser zu vervollständigen, hat es sich für die Erfindung als vorteilhaft erwiesen, Temperaturen bis 140 °C anzuwenden. Hierbei ist also im Rahmen der Erfindung die Zweistufenhydrolyse des MMP-Cyanhydrins mit im Vergleich zum Stand der Technik höherkonzentrierter Schwefelsäure in unterstöchiometrischem bis höchstens stöchiometrischem Verhältnis durchzuführen, wobei im Falle der unterstöchiometrischen Dosierwg in der ersten Hydrolysestufe (Amid-Bildung) bei niedrigerer Temperatur zur Verkürzung der Reaktionsdauer in der zweiten Stufe weitere Schwefelsäure ggf. bis zum Erreichen der stöchiometrischen Obergrenze bei erhöhter Temperatur zugeführt werden kann, um die Umwandlung des Amids zur Säure zu vervollständigen.

Gegenstand der Erfindung ist auch ein verbessertes MHA, das nach einem Verfahren, wie es hierin weiter oben beschrieben ist, erhältlich ist. Das verbesserte MHA kennzeichnet sich erfindungsgemäß dadurch, daß es mehr als 95 Gew.-% MHA-Gesamt als Summe von monomerem MHA, MHA-Dimerem und MHA-Oligomeren (= MHA-Gesamt) sowie zwischen mehr als 0,1 und weniger als 5 Gew.-% Wasser aufweist. Insbesondere hat es sich für die Erfindung als vorteilhaft erwiesen, daß ohne größere Qualitätseinbußen ein MHA erhalten werden kann, welches sich dadurch kennzeichnet, daß es mehr als 98 Gew.-% MHA als Summe von monomerem MHA, MHA-Dimerem und MHA-Oligomeren sowie einen Wassergehalt von zwischen 0,1 und weniger als 2 Gew.-% aufweist und eine kinematische Viskosität von > 100 $mm^2/s$ bei 25 °C. Dabei hat sich überraschenderweise herausgestellt, daß die kinematische Viskosität, nach Cannon-Fenske gemessen, von Hochkonzentrat (also MHA von zumindest 98 Gew.-% Wirkstoffgehalt) nach Lagerung und Verdünnung vergleichbar ist der kinematischen Viskosität eines 88 gew.-%igen Produktes. Trotz eines sich beim Hochkonzentrat nach einer Lagerung von ca. 300 Tagen bei Raumtemperatur einstellenden relativ hohen Dimeren- und Oligomerengehaltes von ca. 50 Gew.-% entspricht bei Verdünnung des abgelagerten Hochkonzentrates mit Wasser auf ca. 88 Gew.-% dessen kinematische Viskosität der des 88 gew.-%igen Handelproduktes, welches in entsprechenden Lagerversuchen lediglich eine Gleichgewichtskonzentration von nur ca. 25 Gew.-% an Dimeren und Oligomeren aufwies. In beiden Fällen, sowohl beim verdünnten Hochkonzentrat als auch beim Handelsprodukt, war der Gleichgewichtszustand erreicht. Diese Tatsache ist sehr überraschend und erweist sich als großer

Vorteil für eine erfindungsgemäß hergestellte Hochkonzentrat-MHA-Variante. Im Hinblick darauf, daß dimere und oligomere Anteile die Viskosität des MHA's im allgemeinen nachteilig für die praktische Verarbeitung beeinträchtigen, war es um so überraschender, daß trotz hoher Ausgangsgehalte beim sogenannten Hochkonzentrat ein leicht pump- und damit transportierbares Gemisch mit günstiger Viskosität erhaltbar ist. Dies hat vielfältige Vorteile, insbesondere führen Viskosität und vor allem der hohe Wirkstoffgehalt dazu, daß das Hochkonzentrat wirtschaftlicher transportiert werden kann, da weniger Wasser transportiert wird, und trotzdem kann am Zielort in der Futtermühle auf handelsübliche Konzentrationen mit Wasser verdünnt werden, ohne ungünstige höhere Viskositäten hinnehmen zu müssen.

Ferner hat es sich im Rahmen der Erfindung herausgestellt, daß ein qualitativ besonders hochwertiges MHA bei geeigneter Führung der Hydrolysereaktion in Kombination mit der erfindungsgemäß einzusetzenden schonenden Eindampfung mit sehr kurzer Verweilzeit erhältlich ist. Das besonders vorteilhaft herstellbare MHA kennzeichnet sich vor allem durch einen Anteil der Summe aus Dimeren und Oligomeren bezogen auf die Gesamtsumme MHA von ≤ 10 Mol %, bevorzugt < 7 Mol %. Dies bedeutet, daß entgegen dem im Stand der Technik verbreiteten Vorurteil, ein hochkonzentriertes MHA erhältlich ist, welches durch einen äußerst geringen Anteil an Dimeren und Oligomeren eine gut geeignete kurzzeitige Transportform darstellt. Für eine längere Transportdauer ist es dann bevorzugt, sich in Abhängigkeit von der Lagerzeit in zunehmendem Maße bildende Dimere und Oligomere durch Zugabe von Wasser und Einwirkung erhöhter Temperatur wieder in monomeres MHA zu überführen.

Weiterhin ist es im Rahmen der Erfindung möglich, das hochkonzentrierte MHA-Produkt zur Herstellung von Tierfuttermittelsupplementen einzusetzen. Dabei zeigt es sich, daß durch Abmischung des MHA-Konzentrats mit Wasser, Methionin und/oder Salzen des MHA's (bevorzugt Ammonium-MHA) (ggf. $NH_3$ zur Erzeugung von $NH_4$-MHA) alle grundsätzlich vom Markt benötigten nutritiven Wertigkeiten ohne Qualitätsverlust herstellbar sind.

Vor allen Dingen hat es sich äußerst überraschend bei Durchführung der Erfindung herausgestellt, daß die Abmischungen nicht nur durch Zugabe von geeigneten Mischungskomponenten wie Wasser, Methionin und/oder Ammonium-MHA ab Austritt des angestrebten MHA-Produkts aus der Eindampfungsstufe zugänglich sind, sondern daß ebenso und weiterhin besonders vorteilhaft für den Fall der Abmischung mit Ammonium-MHA direkt Ammoniak in das MHA-Produkt aus der Eindampfung eingeleitet werden kann. Hierbei wird in Abhängigkeit von der zugefügten Ammoniak-Menge ein gewünschter Anteil des MHA in Ammonium-MHA umgewandelt.

Weiterhin hat es sich besonders vorteilhaft herausgestellt, daß ein praktisch Dimeren- und Oligomeren-freies Produkt auf Basis der Extraktionslösung erhältlich ist, wenn man der Extraktionslösung vor der Eindampfung Ammoniak zusetzt und die beiden entstehenden Phasen in eine organische und eine wäßrige Phase trennt und die wässrige Phase in das Eindampfungsaggregat, welches bevorzugt eine kurze Verweilzeit aufweist, einspeist. Bei dieser Verfahrensweise ergibt sich am Austritt der Eindampfungsstufe ein Gemisch aus MHA und Ammonium-MHA, welches praktisch vollständig Dimeren- und Oligomeren-frei ist. Weiterhin ist es auch denkbar Ammoniak direkt in das Verdampfungsaggregat einzuspeisen. Außerdem kann Ammoniak auch als Strippmedium eingesetzt werden.

In einer erfindungsgemäß besonders bevorzugten Ausführungsform wird das nach einem hierin beschriebenen Verfahren erhältliche MHA, welches mehr als 98 Gew.-% MHA aufweist, gerechnet aus Summe von monomerem MHA, MHA-Dimerem und MHA-Oligomeren, sowie mehr als 0,1 und weniger als 2 Gew.-% Wasser und eine kinematische Viskosität von > 100 $mm^2/s$ bei 25 °C, zur Herstellung von Mischungen für die Tierfuttermittelsupplementierung verwendet, wobei die Verwendung sich dadurch kennzeichnet, daß man eine Mischung mit Methionin herstellt, deren MHA-Gehalt umfassend Monomeres, Dimeres und Oligomere < 80 Gew.-% ist und deren Oligomeren-Gehalt umfassend MHA-Dimeres und Oligomere nach einer Lagerung über 300 Tage bei Raumtemperatur < 25 Mol-% ist, bezogen auf den gesamten Wirkstoffgehalt aus MHA, -Dimerem und -Oligomeren sowie Methionin.

Bei den Mischungen aus MHA und Methionin handelt es sich um Tierfuttermittelsupplemente mit hervorragenden Eigenschaften. Vom Methionin alleine ist bekannt, daß es praktisch in allen organischen Lösungsmitteln schlecht oder sogar unlöslich ist (selbst in Wasser). Erfindungsgemäß hat es besonders überrascht, daß sich Methionin selbst bei Normaltemperatur in MHA gut löst. Ferner ist vom handelsüblichen 88 gew.-%igen MHA bekannt, daß sich im 300-Tage-Lagerungsversuch bei Raumtemperatur eine ca. 25 Mol-%ige Gleichgewichtskonzentration an Dimeren und Oligomeren einstellt. Wie nun bereits oben ausgeführt, weist das hochkonzentrierte MHA bei 300 Tagen Lagerzeit einen deutlich höheren Dimeren- und Oligomeren-Gehalt von ca. 50 Mol-% auf. Bei der Untersuchung von Mischungen aus Methionin und MHA im Lagerversuch hat sich überraschenderweise herausgestellt, daß nach 300 Tagen Lagerzeit in vorteilhafter Weise ein signifikant geringerer Oligomeren- und Dimeren-Anteil vorhanden ist. Dieser beträgt beispielsweise 20 Mol-% nach 300 Tagen, wobei dies noch um die Methionin-Substitution des MHA zu korrigieren ist, was zu einer Veränderung des Wertes auf ca. 21,5 Mol-% führt. Trotzdem ist diese Verbesserung signifikant, da ein niedrigerer Oligomeren-Gehalt grundsätzlich wegen der günstigeren biologischen Wertigkeit des Monomeren erwünscht ist. Selbst eine Verbesserung von "nur" 3,5 Mol-% kann zu einer signifikanten Verbesserung der Wirksamkeit des Produktes in der Tierfuttermittelsupplementierung führen. Insbesondere ist die Tendenz der Wirkungsverbesserung entscheidend. Darüber hinaus ist auch die Kaltlagerstabilität von Mischungen aus MHA und Methionin überraschenderweise deutlich verbessert. Eine Mischung aus 78 Gew.-% MHA und 10 Gew.-% Methionin zeigt bei -20 °C innerhalb von 4 Wochen keinerlei Kristallisationsneigung (es fällt also keine Substanz aus), was überraschend ist, weil

Methionin in anderen Lösungsmitteln unter vergleichbaren Bedingungen sofort ausfällt. Gängige Marktware mit ca. 88 Gew.-% MHA-Gehalt beginnt bereits bei -20 °C nach 3 bis 21 Tagen auszufallen. Damit ermöglichen es die Mischungen aus Methionin und MHA vorteilhafterweise, bei längeren Transporten unter Kaltbedingungen auf Begleitheizungen in gewissem Maße zu verzichten. Auch die Lagerung unter entsprechenden kalten Bedingungen ist damit deutlich einfacher, weil auf kostspielige Zusatzheizungen verzichtet werden kann. Darüber hinaus ist bemerkenswert, daß die Methionin-Zugabe MHA hinsichtlich der biologischen Wertigkeit aufwertet. Dies ist neu und überraschend, weil bis dato keine methioninhaltigen MHA-Flüssig-Formulierungen bekannt waren und die Zur-Verfügung-Stellung solcher Mischungen vom Fachmann aufgrund der nicht zu erwartenden Löslichkeit des Methionins im MHA auch nicht ohne weiteres nahelag. Insbesondere ist eine Mischung aus MHA und Methionin das erste Flüssig-Futtermittelsupplement mit einer biologischen Wertigkeit, die größer als die der einzig bekannten flüssigen Futtermitteladditive auf MHA-Basis ist.

In noch einer weiteren bevorzugten Ausführungsform ist Gegenstand der Erfindung die Verwendung des MHA's , wie es nach einem hierin geschilderten Verfahren erhältlich ist, zur Herstellung von Mischungen für die Tierfuttermittelsupplementierung, wobei sich die Verwendung dadurch kennzeichnet, daß man eine Mischung mit gasförmigem Ammoniak, wässrigem Ammoniak und/oder Ammonium-MHA herstellt, deren MHA-Gehalt umfassend Monomeres, Dimeres und Oligomere < 80 Gew.-% ist und deren Oligomeren-Gehalt umfassend MHA-Dimeres und -Oligomere nach einer Lagerung über 30 Tage bei 40 °C < 25 Mol-% ist, bezogen auf den gesamten Wirkstoffgehalt aus MHA, -Dimerem und -Oligomeren. Hinsichtlich der Lagerstabilität bei Raumtemperatur bzw. bei der Kaltlagerung zeigen auch Gemische aus MHA und Ammonium-MHA erstaunliche Vorteile gegenüber reinem MHA, wobei diese Vorteile ebenso wie bei Mischungen aus MHA und Methionin nicht ohne weiteres vorhersehbar waren. Insbesondere beeinflußt Ammoniak die Bildungsgleichgewichte zwischen Monomeren, Dimeren und Oligomeren günstig.

Im folgenden wird die Erfindung anhand von Beispielen unter Bezugnahme auf die beigefügte Figur eingehender erläutert.

In den Figuren zeigen:

Figur 1 eine schematische Übersicht für einen Teil einer großtechnischen Anlage zur Gewinnung von MHA, wobei zur Vereinfachung lediglich der Aufarbeitungsteil gezeigt wird;

Figur 2 eine Auftragung der kinematischen Viskosität nach Cannon-Fenske für über 300 Tage gelagertes, hochkonzentriertes MHA (ca. 98 Gew.-%) im Vergleich zu einem 88 gew.-%igen Handelsprodukt sowie einer durch Verdünnung aus einem gelagerten Hochkonzentrat mit Wasser auf einen Gehalt von 88 Gew.-% MHA eingestellten MHA-Lösung, bei verschiedenen Temperaturen;

Figur 3 eine Darstellung der Abhängigkeit der MHA- und Dimeren + Oligomeren-Gehalte in Mol-% bei der Lagerung von MHA-Hochkonzentrat (ca. 98 Gew.-%) über 300 Tage bei Raumtemperatur;

Figur 4 eine Darstellung der Abhängigkeit der MHA- und Dimeren + Oligomeren-Gehalte in Mol-% bei der Lagerung von MHA (88 Gew.-%) über 300 Tage bei Raumtemperatur;

Figur 5 eine Darstellung der Abhängigkeit der MHA + Methionin-, Dimeren + Oligomeren- sowie des Methionin-Gehalts in Mol-% bei der Lagerung von MHA-Mischungen aus 78 Gew.-% MHA und 10 Gew.-% Methionin über 300 Tagen bei Raumtemperatur; und

Figur 6 eine Darstellung der Abhängigkeit der MHA + Ammonium-MHA-, Dimeren + Oligomeren-, Ammonium-MHA-Gehalte in Mol-% bei der Lagerung von MHA-Mischungen aus 78 Gew.-% MHA + 10 Gew.-% Ammonium-MHA über 30 Tage bei 40 °C.

Beispiele

Analytische Bestimmungsmethoden und Definitionen

Die Gehalte an MMP-Cyanhydrin, MHA-Amid, MHA-Monomerem bzw. Methionin wurden in den Prozeßlösungen quantitativ per HPLC durch Vergleich mit einem externen Standard (Reinsubstanz) bestimmt.

Der Gehalt an MHA-Gesamt = MHA-Amid (ggf.) + MHA-Monomeres

(= MHA-Ges.) + MHA-(Dimere + Oligomere)

+ Methionin (ggf.)

wurde durch titrimetrische Bestimmung der Thioether-Funktion mit $KBr/KBrO_3$-Maßlösung bestimmt und als Summe der entsprechenden MHA-Monomeräquivalente in [Gew%] bzw. [g] bzw. [mol] bzw. [Mol%] ausgedrückt.

Der Gehalt an MHA-Dimere + MHA-Oligomere (DIM + OLI) wurde durch Berechnung der Differenz aus MHA-Gesamt und MHA-Monomer + (ggf. MHA-Amid und/oder Methionin) ermittelt und als Summe der entsprechenden MHA-Monomeräquivalente in [Gew%] bzw. [g] bzw. [mol] bzw. [Mol%] ausgedrückt.

Der Wassergehalt wurde per Titration nach Karl-Fischer, der MIBK-Gehalt per GC oder Differenzbildung, der Sulfat- bzw. Ammonium-Gehalt per Ionenchromatographie nach Standard-Verfahren bestimmt.

Beispiel 1

Es wurde in einer Reihe von Versuchen untersucht, inwieweit MMP-Cyanhydrin bei Kontakt mit wäßriger Schwefelsäure unter verschiedenen Bedingungen zum MHA-Amid umgesetzt wird.

Durchführung von Versuch 1

In einem 1 l-Dreihalskolben mit Rückflußkühler, Tropftrichter, Innenthermometer und Magnetrührer wurden 141 g (1.03 Mol) 65 % $H_2SO_4$ bei 50 °C vorgelegt und unter Rühren innerhalb von 30 min 138 g (1.0 Mol) 95 % MMP-Cyanhydrin zugetropft. Nach weiteren 15 min Nachreaktionszeit bei dieser Temperatur war kein MMP-Cyanhydrin mehr in der Reaktionslösung nachweisbar.

Durchführung von Versuch 2

Versuch 2 wurde durchgeführt analog Versuch 1 mit 72 g (0.55 Mol) 75 % $H_2SO_4$ und 138 g (1.0 Mol) 95 % MMP-Cyanhydrin. Nach 150 min Nachreaktionszeit waren noch 0.1 % d. Th. MMP-Cyanhydrin nachweisbar.

Durchführung von Versuch 3

In einem 1 l-Vierhalskolben mit Rückflußkühler, zwei Tropf trichtern, einem Innenthermometer und einem Magnetrührer wurden innerhalb von 15 min gleichzeitig 155 g (1.03 Mol) 65 % $H_2SO_4$ und 138 g (1.0 Mol) 95 % MMP-Cyanhydrin bei einer Temperatur von 50 °C eindosiert. Nach weiteren 15 min Nachreaktionszeit bei dieser Temperatur war kein MMP-Cyanhydrin mehr nachweisbar.

Durchführung von Versuch 4 - 11

Die Versuche 4 - 11 wurden analog Versuch 3 mit den in Tab. 1 zusammengefaßten Reaktionsparametern und Ergebnissen durchgeführt.

Tabelle 1

| | Reaktionsbedingungen | | | | | | | |
|---|---|---|---|---|---|---|---|---|
| Vers. Nr. | Mol-Verhältnis MMP-CH:$H_2SO_4$ | $H_2SO_4$-Konz. [Gew.%] | Reakt.-Temp. [°C] | Reaktionszeit [Min] bis 0 % MMP-CH | Reaktionszeit [Min] bis >0,3 % MMP-CH | Zulaufzeit von MMP-CH1) (und $H_2SO_4$)2) [min] | Nachreakt.-Zeit Endprobe [min] | Rest MMP-CH in Mol% |
| 1 | 1:1,03 | 65 | 50 | <15 | - | 30[1)] | 30 | 0 |
| 2 | 1:0,55 | 75 | 50 | >150 | ~60 | 30[1)] | 150 | 0,1 |
| 3 | 1:1,03 | 65 | 50 | <15 | - | 15[2)] | 15 | 0 |
| 4 | 1:0,55 | 75 | 50 | >75 | ~60 | 15[2)] | 75 | 0,15 |
| 5 | 1:0,55 | 75 | 90 | >105 | ~15 | 15[2)] | 105 | 0,07 |
| 6 | 1:1,03 | 65 | 90 | 0 | - | 15[2)] | 15 | 0 |
| 7 | 1:0,55 | 75 | 50 - 90 | ~20 - 30 | ~10 - 15 | 15[2)] | 30 | 0 |
| 8 | 1:0,6 | 75 | 50 | >75 | ~30 - 40 | 15[2)] | 75 | 0,05 |
| 9 | 1:0,65 | 76,4 | 50 | >45 | ~15 | 15[2)] | 45 | 0,14 |
| 10 | 1:0,7 | 78,9 | 50 | 5 - 10 | <5 | 15[2)] | 15 | 0 |
| 11 | 1:1,03 | 75 | 50 | 0 | 0 | 15[2)] | 15 | 0 |

Wie Tab. 1 zeigt, wurde bei den Versuchen 1, 3, 6, 7, 10 und 11 ein vollständiger Umsatz von MMP-CH in der angegebenen Reaktionszeit gefunden.

Die für den vollständigen Umsatz von MMP-CH mit $H_2SO_4$ notwendige Reaktionszeit steigt mit steigendem Molverhältnis an MMP-CH zu $H_2SO_4$ an (Vergleich der Versuche 8 und 11). Die hier praktizierte unterschiedliche Zugabereihenfolge der Einsatzstoffe ([1]) bzw. 2)) hat keine gravierende Auswirkung auf das Reaktionsergebnis (Vergleich der Versuche 1 und 3 bzw. 2 und 4).

Beispiel 2

Durchführung von Versuch 1

In einem 500 ml-Dreihalskolben mit Rückflußkühler, Tropftrichter, Innenthermometer und Magnetrührer wurden 35,8 g (0.35 mol, 0.7 moleq) 96 % $H_2SO_4$ mit Wasser auf eine Schwefelsäurekonzentration von 78.9 Gew% $H_2SO_4$ verdünnt. Innerhalb von 23 min wurden unter Rühren 67,5 g (0.5 mol) 97.2 % MMP-CH bei einer Temperatur von 49 - 56 °C zugetropft. Nach weiteren 20 min Nachreaktionszeit bei ca. 50 °C wurde mittels HPLC-Analyse die vollständige Umsetzung des Cyanhydrins (0 Gew%) zum MHA-Amid (66.2 Gew%) festgestellt. Der Kolbeninhalt wurde mittels 42,3 g Wasser in einen 200 ml-Stahlautoklaven mit Glaseinsatz, Innentemperaturmessung, Manometer und Magnetrührer überführt, so daß die Konzentration der im Reaktionsgemisch vorhandenen wäßrigen Schwefelsäure 44 Gew% betrug.

Nach Verschließen des Autoklaven wurde das Reaktionsgemisch innerhalb von 45 min auf eine Reaktionstemperatur von 140 °C gebracht und noch 120 min bei dieser Temperatur gerührt. Nach Abkühlen und Öffnen des Autoklaven wurden 150,4 g MHA-Hydrolysat erhalten. Per Titration der Schwefeläquivalente mit Bromid/Bromat-Lösung wurde eine Gesamtmenge von 71.5 g (0.48 mol, 95,2 % d. Th. Gesamtausbeute) MHA erhalten. Mittels HPLC-Analyse wurden 57,5 g (76,6 % d. Th.) MHA-Monomeres gefunden. Daraus ergibt sich ein Differenzwert von 14,0 g (18,6 % d. Th.) MHA-(Dimere + Oligomere), jeweils ausgedrückt als MHA-Monomer. MHA-Amid war mittels HPLC nicht mehr nachweisbar.

Durchführung der Versuche 2 - 5

Die Versuche 2 - 5 wurden durchgeführt analog Versuch 1 mit den in Tab. 2 zusammengefaßten Reaktionsparametern und Ergebnissen. Bei Versuch 4 wurde die 1. Stufe in einem 2 l-Glaskolben, die 2. Stufe in einem 2 l-Autoklav, bei Versuch 5 beide Stufen in einem 2 l-Glaskolben durchgeführt.

Tabelle 2

1. Stufe: MHA-Amid-Bildung

| | **Reaktionsbedingungen** | | | | | **Ergebnisse** | | |
|---|---|---|---|---|---|---|---|---|
| **Vers. Nr.** | **97,2 % MMP-CH [g] [mol]** | **96 % $H_2SO_4$ [g]** | **$H_2SO_4$ [moleq]** | **wäßr. $H_2SO_4$ [Gew.%]** | **Temp. [°C]** | **Zeit für · Zugabe · Reaktion [min]** | **Gehalt MHA-Amid [Gew.%]** | **Gehalt MMP-CH [Gew.%]** |
| **1** | 67,5<br>0,5 mol | 35,8 | 0,7 | 78,9 | 49<br>-56 | 23<br>20 | 66,2 | 0 |
| **2** | 67,5<br>0,5 mol | 35,8 | 0,7 | 78,9 | 49<br>-56 | 23<br>20 | 66,2 | 0 |
| **3** | 67,6<br>0,5 mol | 28,1 | 0,55 | 75 | 46<br>-56 | 35<br>20 | 67,8 | 0 |
| **4** | 404<br>3.0 mol | 168,6 | 0,55 | 75 | 45<br>-50 | 35<br>30 | n. b. | n. b. |
| **5** | 269.7<br>2.0 mol | 204.1 | 1.0 | 65 | 50 | 60<br>30 | 43.6<br>+ 6.4<br>MHA | 0 |

Tabelle 2 Fortsetzung

2. Stufe: MHA-Hydrolysat-Bildung

| Vers. Nr. | Reaktionsbedingungen: Wäßr. $H_2SO_4$ [Gew.-%] | Reaktionsbedingungen: Temp. [°C] | Reaktionsbedingungen: Zeit [min] | Ergebnisse [% d. Th.]: MHA-Amid1) | Ergebnisse [% d. Th.]: MHA-Gesamt2) | Ergebnisse [% d. Th.]: MHA-Mono1) | Ergebnisse [% d. Th.]: MHA-Dimeres + Oligomeres3) |
|---|---|---|---|---|---|---|---|
| 1 | 44 | 56 - 140<br>140 | 45<br>120 | 0 | 95,2 | 76.6 | 18.6 |
| 2 | 44 | 56 - 140<br>140 | 65<br>60 | 0 | 97,5 | 78,6 | 18.9 |
| 3 | 44 | 56 - 140<br>140 | 35<br>120 | 3,8 | 99,1 | 76.4 | 18.9 |
| 4 | 24.3 | 50 - 100<br>100<br>100<br>-140<br>140 | 18<br>60<br>57<br>90 | 1.4 | 95.0 | 88.1 | 5.5 |
| 5 | 43 | 50 - 90<br>90 | 15<br>120 | 0.2 | 99.6 | 90,1 | 9.3 |

1) HPLC
2) Bromid/Bromat-Titration
3) Differenzrechnung aus 1) und 2)
n.b. = nicht bestimmt

Beispiel 3

Isolierung von MHA-Hochkonzentrat aus MHA-Hydrolysat mittels Flüssig/Flüssig-Extraktion und Eindampfung der Extraktionslösung

Verfahrensbeschreibung unter Bezugnahme auf Figur 1

In Figur 1 ist der schematische Aufbau der für Beispiel 3 verwendeten Apparatur gezeigt. Diese besteht im wesentlichen aus folgenden Apparaten

| | |
|---|---|
| Extraktionskolonne (001) = | pulsierte Siebbodenkolonne mit 3 m Länge, 2,1 cm Innendurchmesser, 60 Siebböden, beheiztem Doppelmantel |
| Dünnschicht-Verdampfer (002) = | Sambay-Verdampfer mit 0,08 $m^2$ Austauschfläche, beheiztem Doppelmantel |
| Kondensationssystem (003) = | wassergekühlter Glaskühler |
| Auffangbehälter für zurückgeführtes Wasser bzw. (004/005) | Lösungsmittel |

Das aus der MHA-Hydrolysestufe kommende MHA-Hydrolysat, das im wesentlichen besteht aus MHA (Monomer + Dimere + Oligomere + ggf. Amid), $(NH_4)_2SO_4$ und/oder $NH_4HSO_4$ sowie Wasser, wird nach Vorheizung auf die Extraktionstemperatur oberhalb des 40sten Bodens in die Extraktionskolonne 001 eindosiert. Das Lösungsmittel (hier Methylisobutylketon = MIBK) wird ebenfalls vorgeheizt in den Sumpf der Kolonne gepumpt (Gegenstromprinzip). Zusätzlich wird der Kopf der Kolonne mit Waschwasser beaufschlagt. Das im wesentlichen $(NH_4)_2SO_4$ und/oder $NH_4HSO_4$ sowie Wasser enthaltende Raffinat wird am Kolonnensumpf abgezogen. Die im wesentlichen MHA, Lösungsmittel sowie Wasser enthaltende Extraktionslösung wird am Kolonnenkopf abgeführt und anschließend in den Sambay-Verdampfer 002 eingespeist. Dort wird unter Anlegen eines Vakuums und unter zusätzlichem Einblasen von $H_2O$-Dampf, sowie eines $N_2$-Stromes kurz vor dem Ablauf des Verdampfers MIBK und $H_2O$ gemeinsam aus der Extraktionslösung entfernt. Die Verdampfung wurde so gefahren, daß im Sambayablauf < 2 Gew% $H_2O$ nachweisbar waren (Titration nach Karl-Fischer) und das ablaufende MHA-Hochkonzentrat praktisch lösungsmittelfrei war.

Das aus dem Verdampfer 002 kommende Lösungsmittel/Wasser-Gemisch wurde zunächst in 003 kondensiert, zur Trennung in ein Separationsgefaß geführt. Lösungsmittel bzw. Wasser wurden jeweils in einem Auffangbehälter 004 bzw. 005 gesammelt und von da aus in das Extraktionssystem zurückgeführt. Der Sambayablauf wurde auf Raumtemperatur gekühlt in einen Produktauffangbehälter geführt.

Die Bedingungen und Ergebnisse der einzelnen Versuche 1 - 4 sind im folgenden tabellarisch zusammengefaßt.

Die Zusammensetzung der Extraktionslösung wurde unmittelbar nach dem Kopf, die Zusammensetzung der Raffinatlösung unmittelbar nach dem Sumpf der Extraktionskolonne 001 analysiert.

Die Zusammensetzung des MHA-Hochkonzentrats wurde im Sambay-Sumpfablauf unmittelbar nach der Austrittsstelle ermittelt.

Die für die Extraktion eingesetzten MHA-Hydrolysat-Lösungen wurden in einem druckstabilen 400 1-Rührkessel aus 114,7 kg (874 mol) MMP-Cyanhydrin und 85,7 kg (874 mol, 1,00 moleq) $H_2SO_4$ nach den in Beispiel 2, Versuch 5 angegebenen Bedingungen bzw. aus 142,9 kg (1089 mol) MMP-Cyanhydrin und 110,0 kg (1122 mol, 1,03 moleq) $H_2SO_4$ nach Beispiel 2, Versuch 5 bzw. aus 115,3 kg (879 mol) MMP-Cyanhydrin und 47,5 kg (484 mol, 0,55 moleq) $H_2SO_4$ nach Beispiel 2, Versuch 4 hergestellt. Die Roh-Hydrolysatlösungen wurden nach Beendigung der Reaktion jeweils durch Anlegen eines Vakuums von ggf. vorhandenen leichtflüchtigen Nebenprodukten befreit und anschließend analysiert. Die dabei erhaltenen Zusammensetzungen des MHA-Hydrolysats, das zur Extraktion eingesetzt wurde, ist bei den einzelnen Versuchen in Beispiel 3 jeweils angegeben.

Herstellung von MHA-Hochkonzentrat

Versuch 1

Einsatz von MHA-Hydrolysat aus MMP-Cyanhydrin und 1,0 moleq $H_2SO_4$.

Einsatz in Extraktion:
Mengenströme

- MIBK 5 kg/h
- MHA-Hydrolysat 8,4 kg/h
- MHA-Gesamt 3,4 kg/h
- Wasch-$H_2O$ 0,5 kg/h
- MIBK/Hydrolysat 0,6 [-]

Zusammensetzung des MHA-Hydrolysats:
(hergestellt analog Beispiel 2, Versuch 5)

- MHA-Ges: 42 Gew.%; MHA 90 Mol%; Dimere + Oligomere 10 Mol%
- $H_2O$: 28,9 Gew.%
- $SO_4^{2-}$: 25,9 Gew.%

Extraktion (001):
Temperatur: 60 °C (Durchschnitt)
Zusammensetzungen

- der Extraktionslösung:

  MIBK 47 Gew.%
  MHA-Ges. 40 Gew.%
  $H_2O$ 13 Gew.%

- des Raffinats: MHA-Ges. 0,1 Gew.%

Eindampfung (002):
Druck: 100 mbar
Sambay

- Temperatur

  im Heizmantel: 150 °C
  im Kopf: 62 °C
  im Sumpf: n. b.

- Strippdampf: 0,5 kg/h
- Strippgas $N_2$: 100 l/h
- Zusammensetzung des MHA-Hochkonzentrats im Sumpfablauf:

  MHA Ges.: 98 Gew.%; MHA 86 Mol%, Dimere + Oligomere 14 Mol%
  $H_2O$: 2 Gew.%
  MIBK: 40 ppm

Aus dem Sambay-Sumpfablauf wurden ca. 3,5 kg/h MHA-Hochkonzentrat mit der oben genannten Zusammensetzung erhalten.

Versuch 2:

Einsatz von MHA-Hydrolysat aus MMP-Cyanhydrin und 1.03 moleq $H_2SO_4$.
Einsatz in Extraktion:
Mengenströme

- MIBK 3,6 kg/h
- MHA-Hydrolysat 5,38 kg/h
- MHA-Gesamt 2,10 kg/h
- Wasch-$H_2O$ 0,5 kg/h
- MIBK/Hydrolysat 0,67 [-]

Zusammensetzung des MHA-Hydrolysats:
(hergestellt analog Beispiel 2, Versuch 5)

- MHA ges: 39,0 Gew.%; MHA 86,9 Mol%; Dimere + Oligomere 13,1 Mol%
- $H_2O$: ca. 30 Gew.% (Rest)
- $SO_4^{2-}$: 25,4 Gew.%

- $NH_4HSO_4$: 30,4 Gew.%

Extraktion (001):
Temperatur: 54 °C (Durchschnitt)
Zusammensetzungen

- der Extraktionslösung:

  MIBK ≤ 54 Gew.%
  MHA Ges. 34,1 Gew.%
  $H_2O$ 11,9 Gew.%

- des Raffinats:

  MHA-Ges. 0,3 Gew.%
  $SO_4^{2-}$ 30,4 Gew.%
  MIBK 0,17 Gew.%

Eindampfung (002):
Druck: 100 mbar
Sambay

- Temperatur

  im Heizmantel: 150 °C
  im Kopf: 65 °C
  im Sumpf: 92 °C

- Strippdampf: 0,7 kg/h
- Strippgas $N_2$: 100 l/h
- Zusammensetzung des MHA-Hochkonzentrats im Sumpfablauf:

  MHA-Ges.: 99,2 Gew.%; MHA 86,9 Mol%, Dimere+Oligomere 13,1 Mol%
  $H_2O$: 0,8 Gew.%
  $SO_4^{2-}$: 0.43 Gew.%
  MIBK: 28 ppm

Aus dem Sambay-Sumpfablauf wurden ca. 2,1 kg/h MHA-Hochkonzentrat mit der oben genannten Zusammensetzung erhalten.

Versuch 3:

Einsatz von MHA-Hydrolysat aus MMP-Cyanhydrin und 0,55 moleq $H_2SO_4$.
Einsatz in Extraktion:
Mengenströme

- MIBK 3,6 kg/h
- MHA-Hydrolysat 6,55 kg/h
- MHA-Gesamt 2,62 kg/h
- Wasch-$H_2O$ 0,5 kg/h
- MIBK/Hydrolysat 0,55 [-]

Zusammensetzung des MHA-Hydrolysats:
(hergestellt analog Beispiel 2, Versuch 4)

- MHA-Ges.: 40 Gew.%; MHA 94,7 Mol%; Dimere+Oligomere 5,3 Mol%
- $H_2O$: ca. 40,2 Gew.%

- $SO_4^{2-}$: 13,9 Gew.%
- $NH_4^+$: 4,85 Gew.%

Extraktion (001)
Temperatur: 52 °C (Durchschnitt)
Zusammensetzungen

- der Extraktionslösung:

  MIBK ~ 49 Gew.%
  MHA-Ges. 35,8 Gew.%
  $H_2O$ 14,9 Gew.%

- des Raffinats: MHA-Ges. 0,13 Gew.%

Eindampfung (002):
Druck: 100 mbar
Sambay

- Temperatur

  im Heizmantel: 150 °C
  im Kopf: 59 °C
  im Sumpf: 92 °C

- Strippdampf: 0,6 kg/h
- Strippgas $N_2$: 100 l/h
- Zusammensetzung des MHA-Hochkonzentrats im Sumpfablauf:

  MHA-Ges.: 98,8 Gew.%; MHA 95,1 Mol%, Dimere+Oligomere 4,9 Mol%
  $H_2O$: 0,5 Gew.%
  $SO_4^{2-}$: 0.18 Gew.%
  MIBK: 477 ppm

Aus dem Sambay-Sumpfablauf wurden ca. 2,7 kg/h MHA-Hochkonzentrat mit der oben genannten Zusammensetzung erhalten.

Versuch 4:

Einsatz von MHA-Hydrolysat aus MMP-Cyanhydrin und 0,55 moleq $H_2SO_4$.
Einsatz in Extraktion:
Mengenströme

- MIBK 3,6 kg/h
- MHA-Hydrolysat 6,89 kg/h
- MHA-Gesamt 2,76 kg/h
- $H_2O$ 0,5 kg/h
- MIBK/Hydrolysat 0,52 [-]

Zusammensetzung des MHA-Hydrolysats:
(hergestellt analog Beispiel 2, Versuch 4)

- MHA-Ges.: 40 Gew.%; MHA 94,7 Mol%; Dimere + Oligomere 5,3 Mol%
- $H_2O$: 40,2 Gew.%
- $SO_4^{2-}$: 13,9 Gew.%
- $NH_4^+$: 4.85 Gew.%

Extraktion (001):

Temperatur: 52 °C (Durchschnitt)
Zusammensetzungen

- der Extraktionslösung:

  MIBK ca. 49,3 Gew.%
  MHA-Ges. 35,8 Gew.%
  $H_2O$ 14,9 Gew.%

- des Raffinats: MHA-Ges. 0,1 Gew.%

Eindampfung (002):
Druck: 100 mbar
Sambay

- Temperatur

  im Heizmantel: 150 °C
  im Kopf: 59 °C
  im Sumpf: 95 °C

- Strippdampf: 0,6 kg/h
- Strippgas $N_2$: 100 l/h
- Zusammensetzung des MHA-Hochkonzentrats im Sumpfablauf:

  MHA Ges.: 99,0 Gew.%, MHA 94.9 Mol.%, Dimere + Oligomere 5.1 Mol.%
  $H_2O$: 0,2 Gew.%
  $SO_4^{2-}$: 0,16 Gew.%
  MIBK: 482 ppm

Aus dem Sambay-Sumpfablauf wurden ca. 2,8 kg/h MHA-Hochkonzentrat mit der oben genannten Zusammensetzung erhalten.

Wie der Vergleich der Versuche 1 - 4 zeigt, konnte ein MHA-Hochkonzentrat mit ≥ 98 Gew.% MHA-Gesamt und 0,2 bis 2 Gew.% $H_2O$-Gehalt erhalten werden, ohne daß eine Produktschädigung [Vergrößerte MHA-(Dimeren+Oligomeren)-Anteile] eingetreten ist. Dieses überraschende Ergebnis zeigt sich in besonderer Weise in den MHA-Hochkonzentraten aus den Sulfat-ärmeren MHA-Hydrolysatlösungen (Versuch 3 und 4). Hier liegt der MHA-(Dimeren+Oligomeren)-Anteil bei nur 4.9 - 5.1 Mol% im Vergleich zu 13.1 - 14 Mol% bei Versuch 1 und 2. Das Verhältnis der Gewichtsanteile von MHA-Monomerem zur Summe der Dimeren + Oligomeren [= MHA/MHA-(Dim+Oli)] liegt bei ca. 19 bzw. 6.1 - 6.6. In allen hier gezeigten Fällen liegen die MHA-(Dimeren+Oligomeren)-Anteile somit weit unter den üblichen Werten der MHA-Handelsware mit ca. 20 - 25 Mol% MHA-(Dimeren+Oligomeren)-Anteil.

Darüber hinaus ist es gelungen, das Verhältnis der Gewichtsanteile MHA/MHA-(Dim+Oli) gegenüber den im Patent EP 0 142 488 in den Beispielen 1 - 6 angegebenen Werten von 3,3; 3,2; 3,7 (Beispiel 1), 1,8 (Beispiel 2), 3,0 (Beispiel 3), 5,4 (Beispiel 5) und 5,2 (Beispiel 6) entscheidend zu verbessern.

Beispiel 4:

Herstellung eines MHA-Methionin-$H_2O$-Mischproduktes aus MHA-Hochkonzentrat

Versuch 1

Versuch 1 wurde durchgeführt analog Beispiel 3, Versuch 2 mit dem Unterschied, daß der Sambay-Sumpfablauf in einem Rührbehälter mit Methionin und Wasser versetzt wurde und unter Rühren eine homogene Lösung mit 89 Gew.% MHA-Gesamt*) und ca. 10 Gew.% Wasser erzeugt wurde. Dazu wurden insgesamt 25,7 kg des MHA-Hochkonzentrats mit der genannten Zusammensetzung mit 3.2 kg D,L-Methionin (= Met) und 3,2 kg Wasser versetzt und unter Rühren homogenisiert. Das so erzeugte Produkt (32.1 kg) hatte die folgende, analytisch bestimmte Zusammensetzung:

| | |
|---|---|
| MHA Gesamt*) | 89.2 Gew.% ≅ 100.0 Mol% |
| MHA Monomer | 64.8 Gew.% ≅ 72.6 Mol% |
| MHA-(Dimeres + Oligomere) | 15.1 Gew.% ≅ 16.7 Mol % |
| Met | 9.2 Gew.% ≅ 10.7 Mol% |
| $SO_4^{2-}$ | 0.8 Gew.% |
| MIBK | 14 ppm |

*) hier ist MHA-Gesamt= MHA-(Monomer+Dimer+Oligomere)+Met

Versuch 2

Versuch 2 wurde durchgeführt analog Beispiel 3, Versuch 3 mit dem Unterschied, daß der Sambay-Sumpfablauf in einem Rührbehälter mit Methionin und Wasser versetzt wurde und unter Rühren eine homogene Lösung mit 88 Gew.% MHA-Gesamt*) und ca. 11 Gew.% Wasser erzeugt wurde. Dazu wurden insgesamt 27,0 kg des MHA-Hochkonzentrats mit der genannten Zusammensetzung mit 3.5 kg D,L-Methionin und 4,0 kg Wasser versetzt und unter Rühren gelöst. Das so erzeugte Produkt (34,5 kg) hatte die folgende analytisch bestimmte Zusammensetzung:

| | |
|---|---|
| MHA-Gesamt*) | 88,1 Gew.% ≅ 100,0 Mol% |
| MHA Monomer | 73.8 Gew.% ≅ 83,8 Mol% |
| MHA-(Dimeres + Oligomere | 4,3 Gew.% ≅ 4,9 Mol % |
| Met | 10,0 Gew.% ≅ 11,4 Mol% |
| $SO_4^{2-}$ | 0,16 Gew.% ≅ |
| MIBK | 482 ppm |

*) hier ist MHA-Gesamt= MHA-(Monomer+Dimer+Oligomere)+Met

Beispiel 5

Herstellung von MHA-$NH_4$-Salzlösungen aus MHA-Hochkonzentrat

Es wurden 98,3 g MHA-Extraktionslösung mit einem Gehalt von 40 Gew.% (0.26 mol) MHA-Gesamt, 13 Gew.% Wasser und ca. 47 Gew.% MIBK, hergestellt wie in Beispiel 3, Versuch 1, im Wasserstrahlvakuum bei 55 - 70 °C 2.4 Stunden lang eingedampft. Der Rückstand (MHA-Gesamt 99.9 Gew.%) wurde unter Rühren mit 17.7 g (0,26 mol) 25 % wäßriger Ammoniaklösung versetzt. Dabei stieg die Temperatur auf 53 °C. Nach Abkühlen auf Raumtemperatur wurden 57.6 g einer braunen, klaren Flüssigkeit erhalten mit folgender Zusammensetzung:

| | |
|---|---|
| MHA-Gesamt | 68.3 Gew.% |
| MHA-Monomeres | 59.5 Gew.% |
| MHA-Amid | 0.0 Gew.% |
| MHA-(Dimere+Oligomere) | 8.8 Gew.% |
| $NH_4^+$ | 8.1 Gew.% |
| $H_2O$ | 22.4 Gew.% |

Beispiel 6

Herstellung einer MHA-$NH_4$-Salzlösung aus MHA-Extraktionslösung

Es wurden 3502 g MHA-Extraktionslösung mit einem Gehalt von 35.8 Gew.% MHA-Gesamt, 14,9 Gew.% $H_2O$ und ca. 49 Gew.% MIBK, hergestellt wie in Beispiel 3, Versuch 4 in einem Eisbad auf 5 °C abgekühlt. In diese Lösung wurden innerhalb von 3.5 h bei einer Temperatur von 5 - 21 °C 300 g (17.6 mol) gasförmiger Ammoniak eingeleitet. Dabei bildeten sich zwei flüssige Phasen. Die schwerere Phase (2202 g) wurde abgetrennt und durch Einleiten von Wasserdampf bei 60 °C und 100 mbar Druck von restlichem MIBK befreit. Der Rückstand wurde im Wasserstrahlvakuum bei 50 °C (2 h) weiter aufkonzentriert, wobei eine ölige braungelbe Flüssigkeit (1583 g) erhalten wurde mit der folgenden Zusammensetzung:

| | | |
|---|---|---|
| MHA-Gesamt | | 81.2 Gew.% (≅ 103 % d. Th.) |

(fortgesetzt)

| | | |
|---|---|---|
| incl.: | - MHA-Monomeres | 79.3 Gew.% |
| | - MHA-Amid | 1.9 Gew.% |
| | - MHA-(Dimere + Oligomere) | 0.0 Gew.% |
| $NH_4^+$ | | 9.0 Gew.% |
| Wasser | | 9.8 Gew.% |

Nach Verdünnen mit 230 g Wasser und 20 g 25 %iger Ammoniaklösung wurde eine hellbraune leichtbewegliche Flüssigkeit (1743 g) mit folgender Zusammensetzung erhalten:

| | | |
|---|---|---|
| MHA-Gesamt | | 70.0 Gew.% |
| incl.: | -MHA-Monomeres | 68.3 Gew.% |
| | - MHA-Amid | 1.7 Gew.% |
| | - MHA-(Dimere + Oligomere) | 0.0 Gew.% |
| $NH_4^+$ | | 8.1 Gew.% |
| $H_2O$ | | 20.0 Gew.% |

Überraschenderweise konnten nach dieser Behandlung die unerwünschten MHA-(Dimere+Oligomere) in der Produktlösung nicht mehr nachgewiesen werden. Lediglich ein geringfügiger Gehalt an MHA-Amid war als einzige Nebenkomponente enthalten.

Beispiel 7

Herstellung eines MHA-$MHANH_4$-$H_2O$-Mischproduktes aus MHA-Extraktionslösung

Versuch 1

Unter den im wesentlichen unter Beispiel 3, Versuch 2 angegebenen Bedingungen wurden 10 kg MHA-Extraktionslösung mit einem Gehalt von 39,7 Gew.-% MHA-Gesamt, ca. 12 Gew.-% $H_2O$ und ca. 48 Gew.-% MIBK über den Sambay-Verdampfer eingedampft. Die ablaufende Lösung wurde mit insgesamt 0,636 kg (3.06 mol) einer 8,2 %igen wäßrigen $NH_3$-Lösung verdünnt und gleichmäßig gemischt.
Dabei wurde eine ölige, braungelbe Flüssigkeit (4,60 kg) erhalten (pH ~ 2,3) mit der folgenden Zusammensetzung:

| | | |
|---|---|---|
| MHA-Gesamt | | 86,2 Gew.-% (≙ 100 % d. Th.) |
| incl.: | - MHA-Monomeres | 74,3 Gew.-% |
| | - MHA-Amid | 0,0 Gew.-% |
| | - MHA-(Dimere + Oligomere) | 11,9 Gew.-% |
| $NH_4^+$ | | 1,2 Gew.-% |
| $SO_4^{2-}$ | | 0,22 Gew.-% |
| $H_2O$ | | 13,5 Gew.-% |
| $MHANH_4$ * | | 10,75 Gew.-% |

* berechneter Wert für MHA-$NH_4$-Anteil (≙ 11,2 Mol% als Monomeres, Dimeres und Oligomeres)

Versuch 2

Unter den im wesentlichen unter Beispiel 3, Versuch 2 angegebenen Bedingungen wurden 10 kg MHA-Extraktionslösung mit einem Gehalt von 35,1 Gew.-% MHA-Gesamt, ca. 12,7 Gew.-% $H_2O$ und ca. 52 Gew.-% MIBK über den Sambay-Verdampfer eingedampft. Die ablaufende Lösung wurde mit insgesamt 0,45 kg (4,23 mol) einer 16,0 %igen wäßrigen $NH_3$-Lösung verdünnt und gleichmäßig gemischt.
Dabei wurde eine ölige, braungelbe Flüssigkeit (3,96 kg) vom pH ca. 2,7 erhalten mit der folgenden Zusammensetzung:

| | | |
|---|---|---|
| MHA-Gesamt | | 88,6 Gew.-% (≙ 100 % d. Th.) |

(fortgesetzt)

| | | |
|---|---|---|
| incl.: | - MHA-Monomeres<br>- MHA-Amid<br>- MHA-(Dimere + Oligomere) | 76,9 Gew.-%<br>0,0 Gew.-%<br>11,7 Gew.-% |
| $NH_4^+$ | | 1,94 Gew.-% |
| $SO_4^{2-}$ | | 0,15 Gew.-% |
| $H_2O$ | | 9,8 Gew.-% |
| $MHANH_4$ * | | 17,74 Gew.-% |

* berechneter Wert für MHA-$NH_4$-Anteil (≙ 7,7 Mol% als Monomeres, Dimeres und Oligomeres)

Beispiel 8

Bestimmung der Viskositäten nach Cannon Fenske:

Es wurden, wie am besten in Fig. 2 zu sehen ist, die kinematischen Viskositäten mit einem Viskosimeter des Typs Cannon Fenske opaque nach der Methode ISO 3105-1976 in Abhängigkeit von der Temperatur bestimmt für folgende MHA-Qualitäten:

- MHA 98 %, hergestellt nach Beispiel 3, Versuch 2 und Lagerung bei Raumtemperatur für > 300 Tage, entsprechend der Kurve mit Bezugszeichen 1 in Figur 2,
- MHA 88 % aus MHA 98 %, hergestellt nach Beispiel 3, Versuch 2, Lagerung bei Raumtemperatur für > 300 Tage und anschließender Verdünnung auf 88 %, entsprechend der Kurve mit Bezugszeichen 2 in Figur 2, und
- MHA 88 % übliche Handelsware, entsprechend der Kurve mit Bezugszeichen 3 in Figur 2.

Trotz relativ hoher Dimeren- und Oligomeren-Gehalte direkt nach Verdünnung mit Wasser entsprechen die Viskositäten des dadurch erzeugten MHA 88 aus 98 (2) denen der Handelsware MHA 88 (3).

Beispiel 9

Lagerversuche von MHA-Produkten

Die unter Figur 3 bis Figur 6 genannten Produkte wurden jeweils in einem geschlossenen Glasgefäß ohne Rühren bei den dort angegebenen Temperaturen über einen Zeitraum von bis zu 310 Tagen gelagert. In regelmäßigen Abständen wurden Proben entnommen und der Gehalt an MHA-Gesamt, MHA-Monomeren, MHA-(Dimeren + Oligomeren) sowie ggf. Met bestimmt (vgl. o. a. Methoden).

Figur 3:

MHA-Hochkonzentrat, das nach Beispiel 3, Versuch 2 hergestellt wurde, weist nach ca. 3 Monaten Lagerung bei Raumtemperatur ein eingestelltes Gleichgewicht auf von
47 Mol-% MHA-Monomeren (1) und
53 Mol-% MHA-(Dimeren + Oligomeren) (2).

Figur 4:

MHA 88, das analog Beispiel 3, Versuch 2 und anschließender Verdünnung mit Wasser auf 88 % MHA-Gehalt hergestellt wurde, weist nach ca. 3 Monaten Lagerung bei Raumtemperatur ein eingestelltes Gleichgewicht auf von
74 Mol-% MHA-Monomeren (1)
26 Mol-% MHA-Dimeren + Oligomeren (2).

Dieses Verhältnis wurde auch in handelsüblicher Ware gefunden.

Figur 5:

MHA 78 + Met 10, das nach Beispiel 4, Versuch 1 hergestellt wurde, weist nach ca. 3 Monaten Lagerung bei

Raumtemperatur ein eingestelltes Gleichgewicht auf von
80 Mol-% MHA-Monomeres + Met (1)
20 Mol-% MHA-Dimeren + Oligomeren (2)

Figur 6:

MHA 78 + $MHANH_4$ 10, das nach Beispiel 7 hergestellt wurde, weist bereits nach 14 Tagen Lagerung bei 40 °C ein eingestelltes Gleichgewicht auf von
80 Mol-% MHA-Monomeren (1) und
20 Mol-% MHA-Dimeren + Oligomeren (2).

Der Vergleich von Figur 3, 4, 5 und 6 zeigt, daß Mischungsprodukte wie das MHA 78 + Met 10 bzw. MHA 78 + $MHANH_4$ 10 deutlich günstigere (Dimeren + Oligomeren)-Anteile aufweisen als handelsübliches MHA 88 nach längerer Lagerung. Bei MHA 78 + $MHANH_4$ 10 konnte der Gleichgewichtszustand (bedingt durch höhere Temperatur) bereits deutlich eher erreicht werden (Figur 6) als in den anderen gezeigten Fällen.

**Patentansprüche**

**1.** Verfahren zur Gewinnung von 2-Hydroxy-4-methylthiobuttersäure (MHA), bei dem das MHA aus einem Reaktionsgemisch isoliert wird, welches durch Anlagerung von Blausäure (HCN) an Methylmercaptopropionaldehyd (MMP) und Hydrolyse des dabei erhaltenen Methylmercaptopropionaldehyd-Cyanhydrins (MMP-CH) mit Schwefelsäure erhalten wird, wobei das Reaktionsgemisch mit einem im wesentlichen mit Wasser nicht mischbaren organischen Lösungsmittel in einem Flüssig/Flüssig-Extraktionssystem in Kontakt gebracht wird, um eine Extraktionslösung zu bilden, die das Lösungsmittel und aus dem Reaktionsgemisch überführtes MHA aufweist, und das MHA als Extrakt aus dieser Extraktionslösung durch Eindampfen gewonnen wird,
**dadurch gekennzeichnet,**
daß die Eindampfung so geführt wird, daß der verbleibende Extrakt weniger als 4 Gew.-% Wasser aufweist.

**2.** Verfahren nach Anspruch 1,
**dadurch gekennzeichnet,**
daß das organische Lösungsmittel bei der Eindampfung mit einem Dünnschichtverdampfer, Fallfilmverdampfer, Kurzwegverdampfer und/oder einer Strippstufe oder unter Mithilfe eines solchen Aggregats entfernt wird.

**3.** Verfahren nach Anspruch 1 oder 2,
**dadurch gekennzeichnet,**
daß der Druck während der Eindampfung nicht mehr als 600 mbar beträgt.

**4.** Verfahren nach Anspruch 1 bis 3,
**dadurch gekennzeichnet,**
daß die Temperatur des MHA als Extrakt während der Eindampfung nicht wesentlich höher als 150 °C ist.

**5.** Verfahren nach Anspruch 4,
**dadurch gekennzeichnet,**
daß die Temperatur des MHA als Extrakt unmittelbar am Austritt der Eindampfung zwischen 30 und 100 °C beträgt.

**6.** Verfahren nach einem der vorhergehenden Ansprüche,
**dadurch gekennzeichnet,**
daß die Verweilzeit des verbleibenden Extrakts in der Eindampfung nicht länger als 1,5 h beträgt.

**7.** Verfahren nach einem der vorhergehenden Ansprüche,
**dadurch gekennzeichnet,**
daß die Hydrolyse des MMP-CH so geführt wird, daß in einer ersten Stufe das MMP-CH mit 60 - 85 %iger Schwefelsäure im Molverhältnis MMP-CH: $H_2SO_4$ von 1:0,5 bis 1:1,05 bei Temperaturen von 30 - 90 °C unter Erhalt von im wesentlichen MHA-Amid hydrolysiert und dieses in einer zweiten Stufe unter Zugabe von Wasser ohne weitere Zugabe von $H_2SO_4$ bei Temperaturen bis 140 °C hydrolysiert wird.

**8.** MHA, erhältlich nach einem in den vorhergehenden Ansprüchen beschriebenen Verfahren, aufweisend mehr als 95 Gew.-% MHA, insbesondere mehr als 98 Gew.-%, gerechnet als Summe von monomerem MHA, MHA-Dimerem

und MHA-Oligomeren, sowie mehr als 0,1 und weniger als 2 Gew.-% Wasser und eine kinematische Viskosität von > 100 $mm^2/s$ bei 25 °C.

9. Verwendung des MHA's nach Anspruch 8 zur Herstellung von Mischungen für die Tierfuttermittelsupplementierung,
**dadurch gekennzeichnet,**
daß man eine Mischung mit Methionin herstellt, deren MHA-Gehalt umfassend Monomeres, Dimeres und Oligomere < 80 Gew.-% ist und deren Oligomerengehalt umfassend MHA-Dimeres und -Oligomere nach einer Lagerung über 300 Tage bei Raumtemperatur < 25 Mol% ist, bezogen auf den gesamten Wirkstoffgehalt aus MHA, -Dimerem und -Oligomeren sowie Methionin.

10. Verwendung des MHA's nach Anspruch 8 zur Herstellung von Mischungen für die Tierfuttermittelsupplementierung,
**dadurch gekennzeichnet,**
daß man eine Mischung mit gasförmigem Ammoniak, wässrigem Ammoniak und/oder Ammonium-MHA herstellt, deren MHA-Gehalt umfassend Monomeres < 80 Gew.-% ist und deren Oligomerengehalt umfassend MHA-Dimeres und -Oligomere nach einer Lagerung über 30 Tage bei 40 °C < 25 Mol% ist, bezogen auf den gesamten Wirkstoffgehalt aus MHA, -Dimeren und -Oligomeren.

## Claims

1. Process for the recovery of 2-hydroxy-4-methylthiobutyric acid (MHA), wherein the MHA is isolated from a reaction mixture which is obtained by addition of hydrocyanic acid (HCN) to methylmercaptopropionaldehyde (MMP) and hydrolysis with sulfuric acid of the methylmercaptopropionaldehyde cyanohydrin (MMP-CH) thus obtained,
the reaction mixture being brought into contact, in a liquid/liquid extraction system, with an organic solvent which is substantially immiscible with water, in order to form an extracting solution which contains the solvent and MHA transferred out of the reaction mixture, and the MHA being recovered as extract from this extracting solution by evaporation,
characterised in that
the evaporation is carried out in such a way that the extract remaining contains less than 4 wt.% water.

2. Process according to claim 1,
characterised in that
the organic solvent is removed during the evaporation by means of a film evaporator, downflow evaporator, short-path evaporator and/or a stripping step or with the assistance of such a unit.

3. Process according to claim 1 or 2,
characterised in that
the pressure during the evaporation is not more than 600 mbar.

4. Process according to claims 1 to 3,
characterised in that
the temperature of the MHA as extract during the evaporation is not substantially higher than 150°C.

5. Process according to claim 4,
characterised in that
the temperature of the MHA as extract immediately at the outlet from the evaporation is between 30°C and 100°C.

6. Process according to one of the preceding claims,
characterised in that
the residence time of the extract remaining in the evaporation is not longer than 1.5 h.

7. Process according to one of the preceding claims,
characterised in that
the hydrolysis of the MMP-CH is carried out in such a way that, in a first step, the MMP-CH is hydrolysed with 60% to 85% sulfuric acid in the molar ratio MMP-CH: $H_2SO_4$ of 1:0.5 to 1:1.05 at temperatures of from 30°C to 90°C, with substantially MHA amide being obtained and this, in a second stage, is hydrolysed by addition of water without

further addition of $H_2SO_4$ at temperatures of up to 140°C.

8. MHA, obtainable by a process described in the preceding claims, containing more than 95 wt.% MHA, in particular more than 98 wt.%, calculated as the sum of monomeric MHA, MHA dimer and MHA oligomers, as well as more than 0.1 and less than 2 wt.% water and having a kinematic viscosity of > 100 $mm^2/s$ at 25°C.

9. Use of the MHA according to claim 8 for the preparation of mixtures for animal feed supplementation, characterised in that
a mixture with methionine is prepared, the MHA content of which, including monomer, dimer and oligomers, is < 80 wt.% and the oligomer content of which, including MHA dimer and MHA oligomers, after storage for 300 days at room temperature is < 25 mol.%, based on the total content of active ingredients consisting of MHA, MHA dimer and MHA oligomers as well as methionine.

10. Use of the MHA according to claim 8 for the preparation of mixtures for animal feed supplementation, characterised in that
a mixture with gaseous ammonia, aqueous ammonia and/or ammonia-MHA is prepared, the MHA content of which, including monomer, is < 80 wt.% and the oligomer content of which, including MHA dimer and MHA oligomers, after storage for 30 days at 40°C, is < 25 mol.%, based on the total content of active ingredients consisting of MHA, MHA dimers and MHA oligomers.

**Revendications**

1. Procédé pour l'obtention d'acide 2-hydroxy-4-méthylthiobutyrique (MHA), dans lequel le MHA est isolé à partir d'un mélange réactionnel qui est obtenu par addition d'acide cyanhydrique (HCN) à du méthylmercaptopropionaldéhyde (MMP) et hydrolyse de la cyanhydrine de méthylmercaptopropionaldéhyde (MMP-CH) obtenue avec de l'acide sulfurique, le mélange réactionnel étant mis en contact avec un solvant organique essentiellement non miscible à l'eau, dans un système d'extraction liquide/liquide, afin de former une solution d'extraction qui contient le solvant et le MHA transféré à partir du mélange réactionnel, le MHA étant obtenu sous forme d'extrait à partir de cette solution d'extraction par concentration par évaporation, caractérisé en ce que la concentration par évaporation est réalisée de telle manière que l'extrait résiduel contient moins de 4 % en poids d'eau.

2. Procédé selon la revendication 1, caractérisé en ce que le solvant organique est éliminé au cours de la concentration par évaporation avec un évaporateur à couche mince, un évaporateur à film tombant, un évaporateur à vide élevé et/ou un dispositif de stripage, ou à l'aide d'un tel appareil.

3. Procédé selon la revendication 1 ou 2, caractérisé en ce que la pression, au cours de la concentration par évaporation, n'est pas supérieure à 600 mbars.

4. Procédé selon la revendication 1 à 3, caractérisé en ce que la température du MHA en tant qu'extrait, au cours de la concentration par évaporation, n'est pas essentiellement supérieure à 150 °C.

5. Procédé selon la revendication 4, caractérisé en ce que la température du MHA en tant qu'extrait, immédiatement à la sortie de la concentration par évaporation est comprise entre 30 et 100 °C.

6. Procédé selon une quelconque des revendications précédentes, caractérisé en ce que le temps de séjour de l'extrait résiduel dans la concentration par évaporation n'est pas supérieur à 1,5 h.

7. Procédé selon l'une quelconque des revendications précédentes, caractérisé en ce que l'hydrolyse du MMP-CH est réalisée de telle manière qu'on hydrolyse, au cours d'une première étape, le MMP-CH avec de l'acide sulfurique à 60-85 %, dans un rapport molaire MMP-CH : $H_2SO_4$ compris entre 1:0,5 et 1:1,05, à des températures comprises entre 30 - 90 °C, en obtenant essentiellement de l'amide de MHA et en ce que celui-ci est hydrolysé au cours d'une deuxième étape par addition d'eau, sans addition supplémentaire de $H_2SO_4$, à des températures allant jusqu'à 140 °C.

8. MHA, obtenu selon l'un quelconque des procédés décrits dans les revendications précédentes, présentant plus de 95 % en poids de MHA, en particulier plus de 98 % en poids, calculé sous forme de la somme des monomères de MHA, des dimères de MHA et des oligomères de MHA, ainsi que plus de 0,1 et moins de 2 % en poids d'eau

et une viscosité cinématique supérieure à 100 $mm^2/s$ à 25°C.

**9.** Utilisation du MHA selon la revendication 8 pour la préparation de mélanges pour les suppléments pour aliments pour animaux, caractérisée en ce qu'on réalise un mélange avec de la méthionine dont la teneur en MHA, comprenant les monomères, les dimères et les oligomères, est inférieure à 80 % en poids et dont la teneur en oligomères, comprenant les dimères de MHA et les oligomères de MHA, après un stockage pendant 300 jours à température ambiante est inférieure 25 mole-% par rapport à la teneur totale en substance active, constituée par le MHA, les dimères de MHA, les oligomères de MHA et la méthionine.

**10.** Utilisation du MHA selon la revendication 8 pour la préparation de mélanges pour les suppléments pour aliments pour animaux, caractérisée en ce qu'on prépare un mélange avec de l'ammoniac gazeux, de l'ammoniaque aqueux et/ou de l'ammonium-MHA, dont la teneur en MHA, comprenant les monomères, est inférieure à 80 % en poids et dont la teneur en oligomères, comprenant les dimères de MHA et les oligomères de MHA, après un stockage pendant 30 jours à 40 °C est inférieure à 25 mole-%, par rapport à la teneur totale en substance active, constituée par le MHA, les dimères de MHA et les oligomères de MHA.

Figur 1

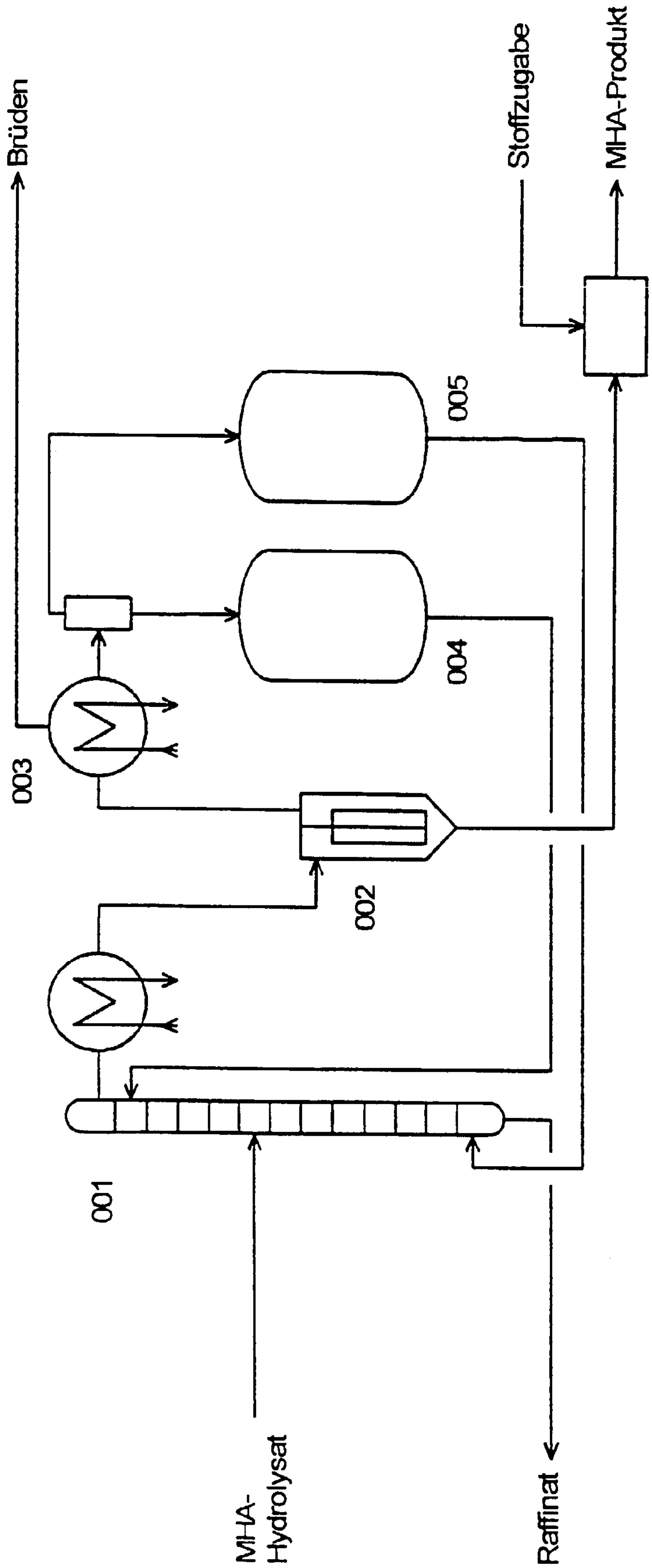
Brüden
Stoffzugabe
MHA-Produkt
005
004
003
002
001
MHA-
Hydrolysat
Raffinat

Figur 2

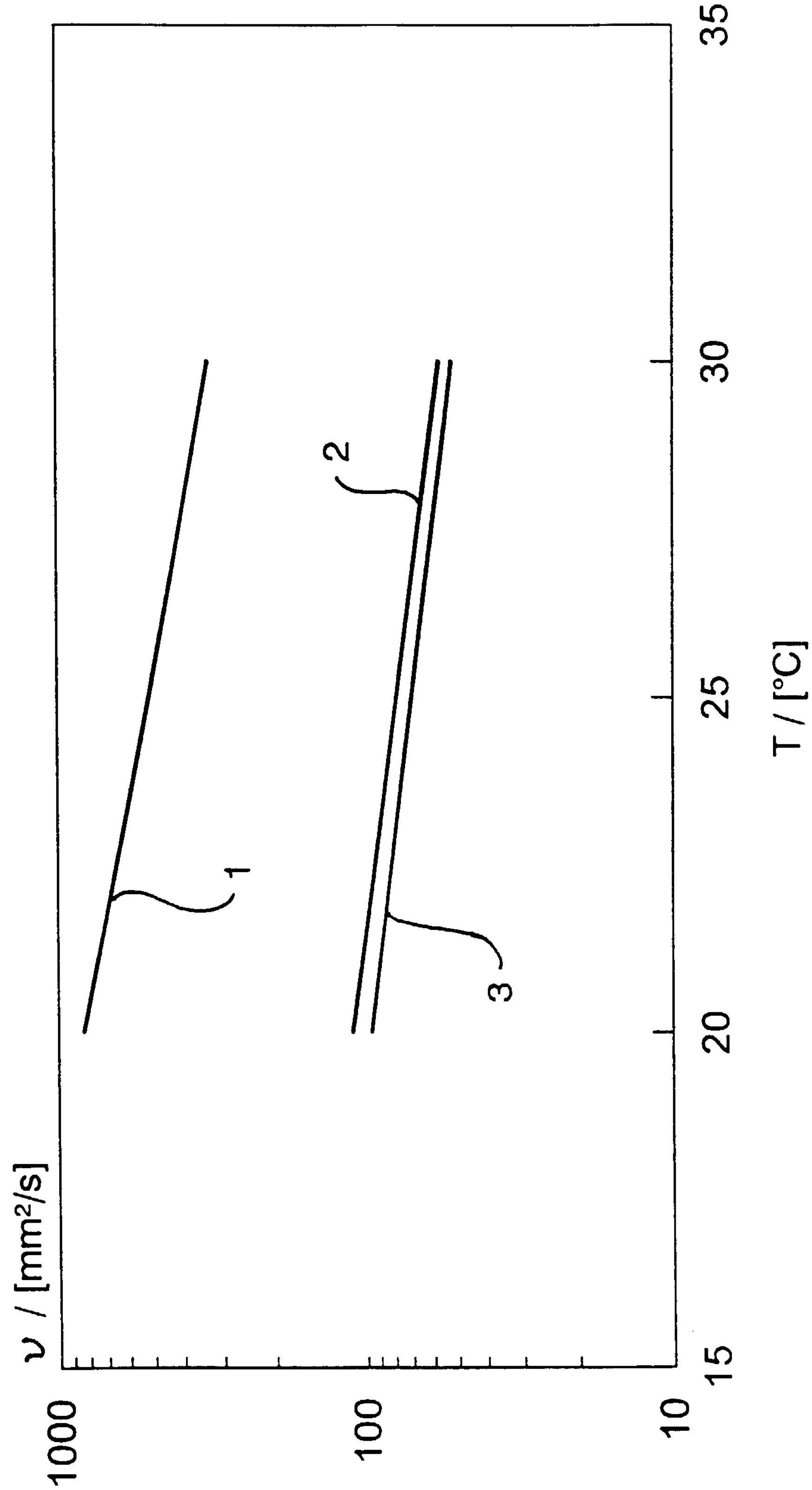

Figur 3

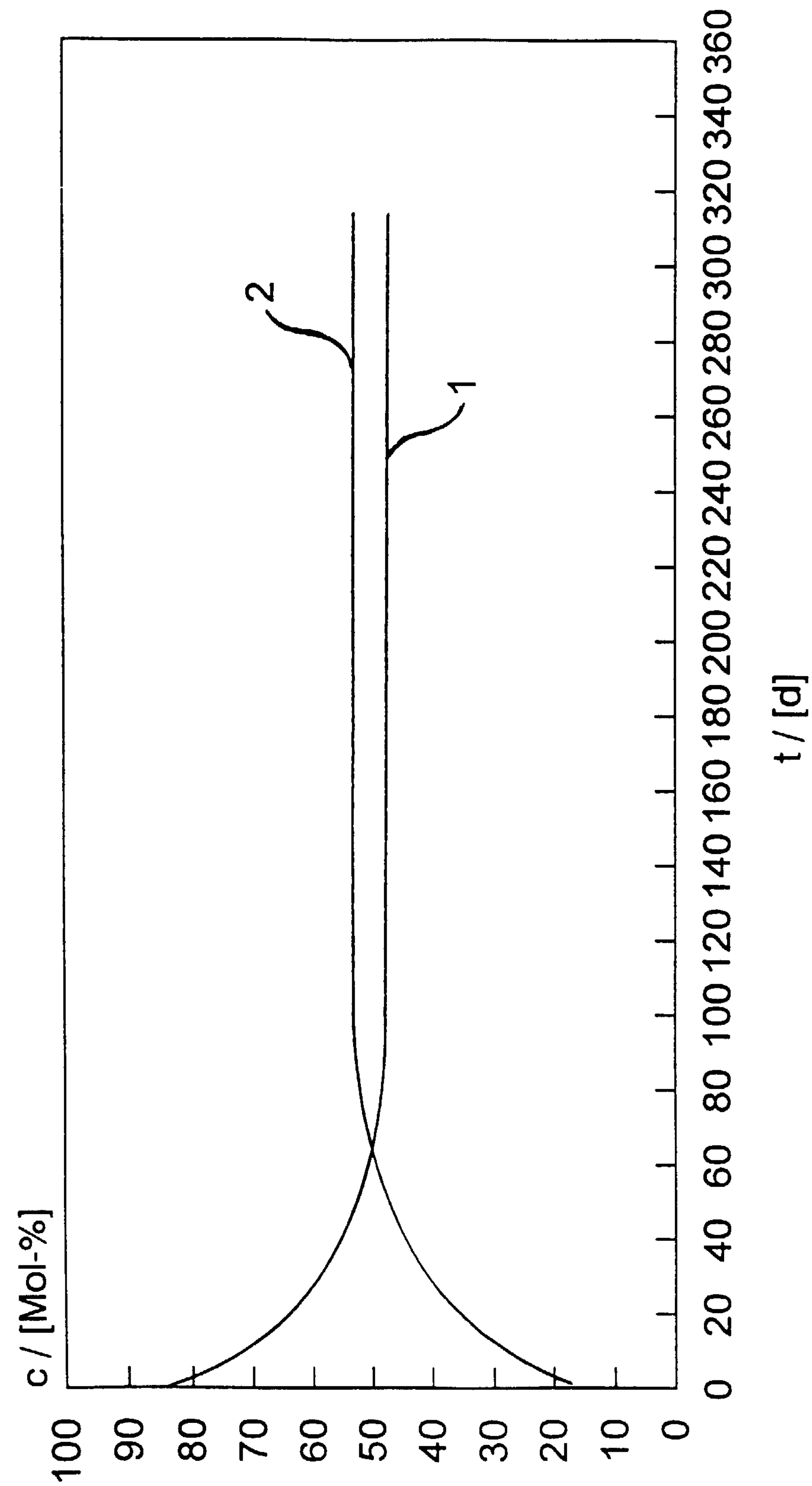
c / [Mol-%]
100
90
80
70
60
50
40
30
20
10
0
0 20 40 60 80 100 120 140 160 180 200 220 240 260 280 300 320 340 360
t / [d]
1
2

Figur 4

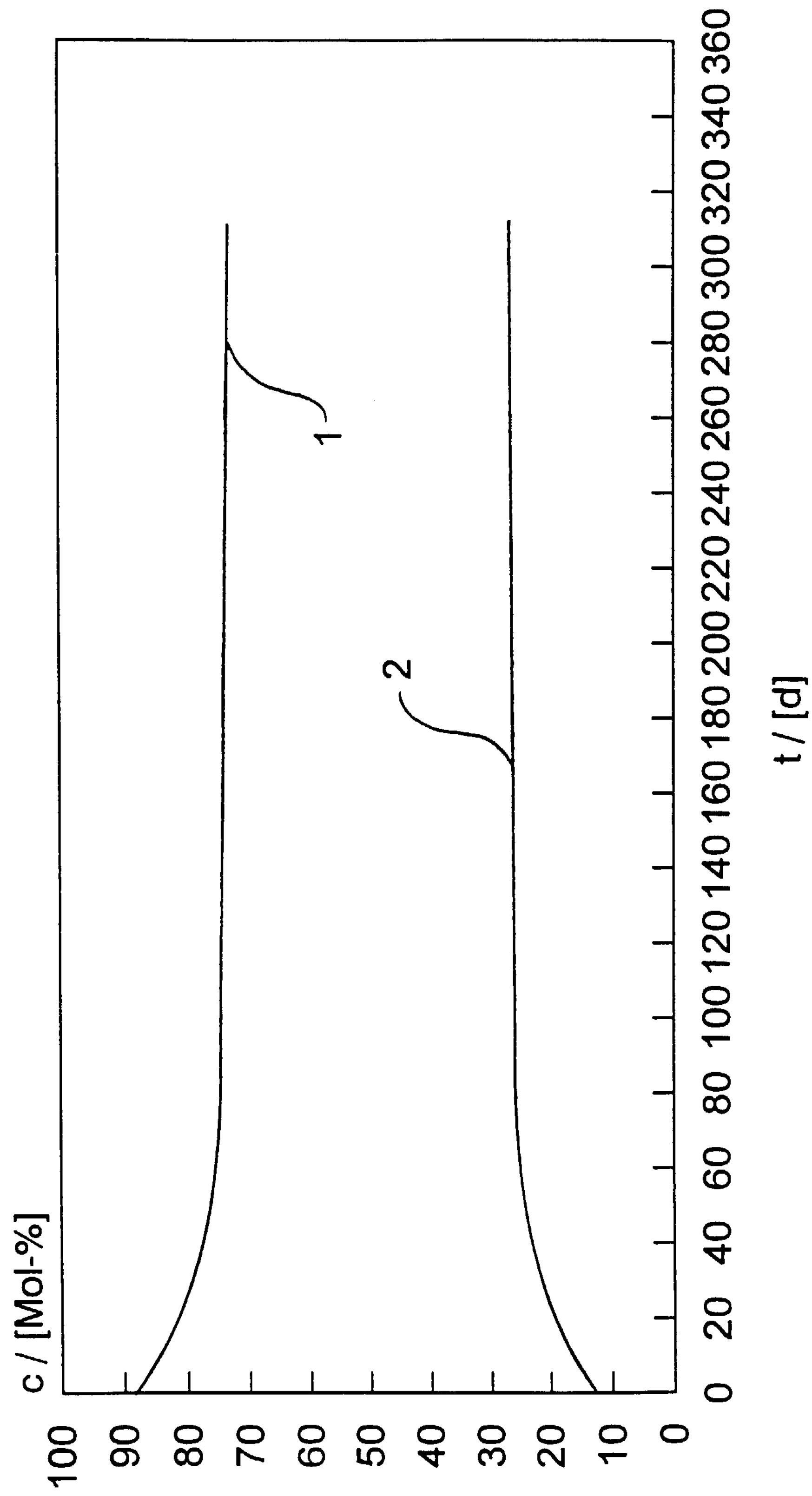
c / [Mol-%]
100
90
80
70
60
50
40
30
20
10
0
0 20 40 60 80 100 120 140 160 180 200 220 240 260 280 300 320 340 360
t / [d]
1
2

Figur 5

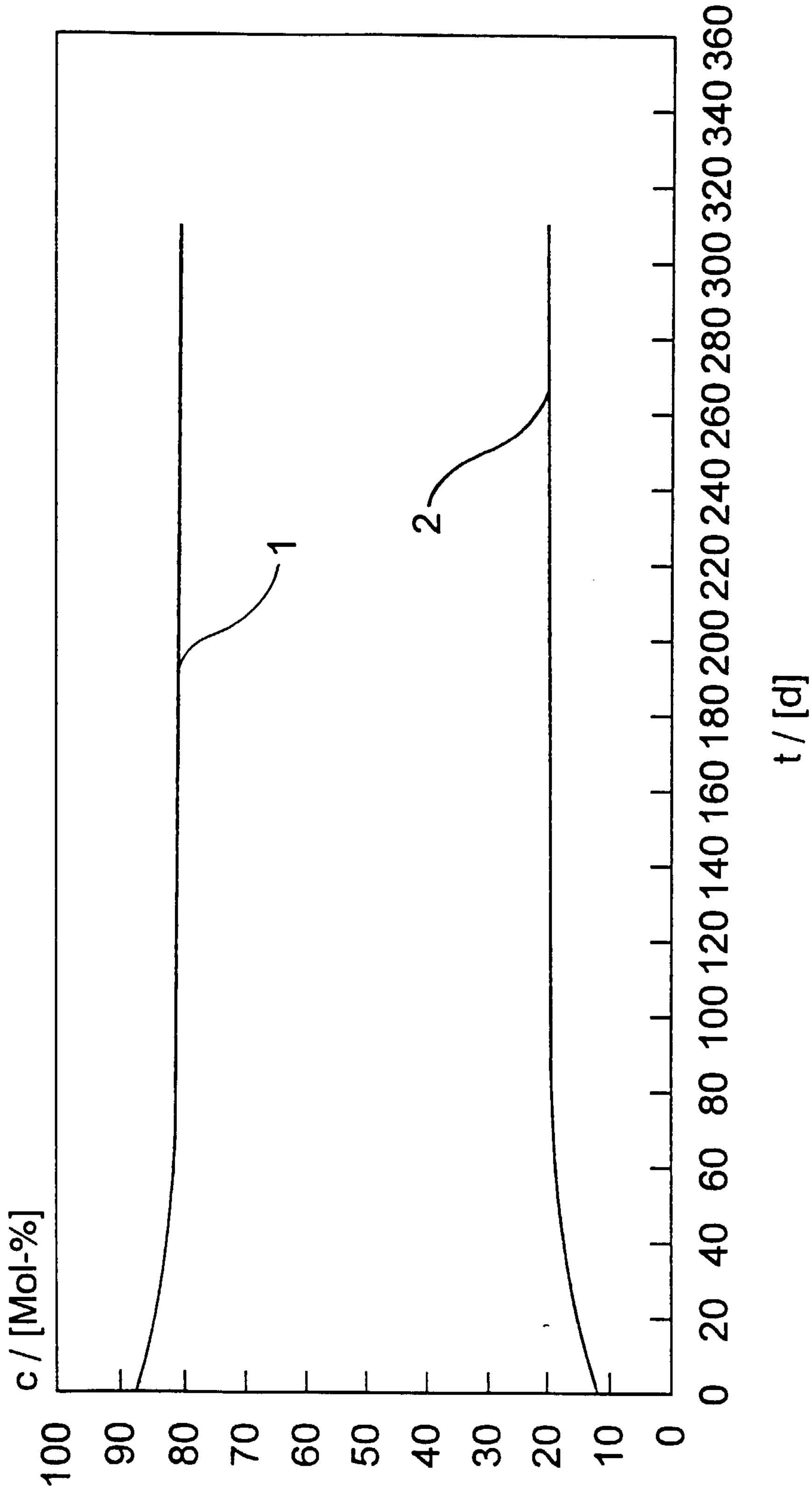
c / [Mol-%]
100
90
80
70
60
50
40
30
20
10
0
0
20
40
60
80
100
120
140
160
180
200
220
240
260
280
300
320
340
360
t / [d]
1
2

Figur 6

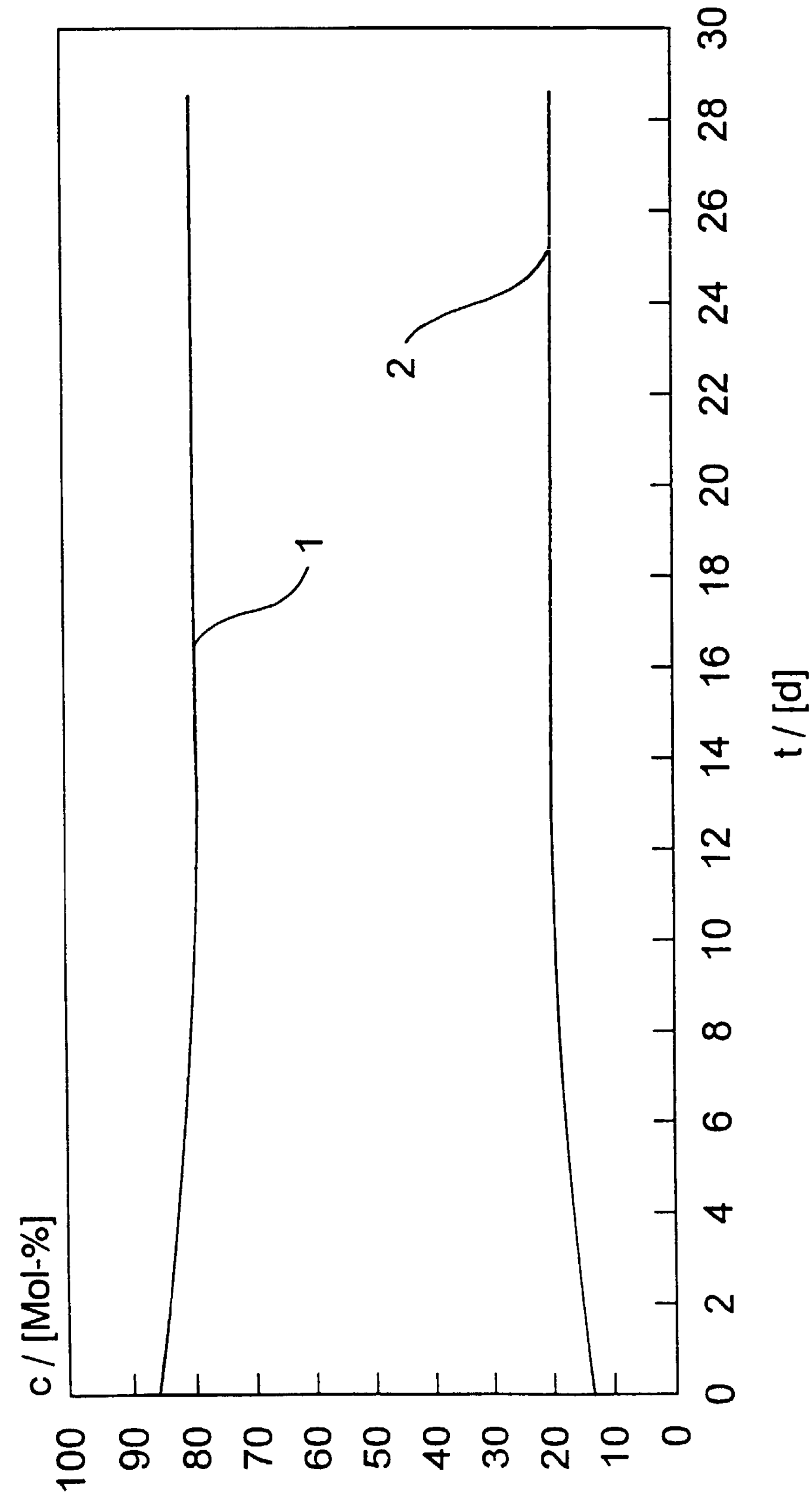
c / [Mol-%]
100
90
80
70
60
50
40
30
20
10
0
0
2
4
6
8
10
12
14
16
18
20
22
24
26
28
30
t / [d]
1
2